(19) Europäisches Patentamt
European Patent Office
Office européen des brevets

(11) **EP 3 899 701 B1**

(12) **FASCICULE DE BREVET EUROPEEN**

(45) Date de publication et mention
de la délivrance du brevet:
**10.01.2024   Bulletin 2024/02**

(21) Numéro de dépôt: **19836807.8**

(22) Date de dépôt: **23.12.2019**

(51) Classification Internationale des Brevets (IPC):
**G06F 3/041** *(2006.01)*      **A61B 5/16** *(2006.01)*
**A61B 5/00** *(2006.01)*

(52) Classification Coopérative des Brevets (CPC):
**G06F 3/0416; A61B 5/162; A61B 5/6898**

(86) Numéro de dépôt international:
**PCT/EP2019/086899**

(87) Numéro de publication internationale:
**WO 2020/128088 (25.06.2020 Gazette 2020/26)**

(54) **MESURE TEMPORELLE A HAUTE PRECISION D'EVENEMENTS VIBRO-ACOUSTIQUES EN SYNCHRONISATION AVEC UN SIGNAL SONORE SUR DISPOSITIF A ECRAN TACTILE**

HOCHGENAUE ZEITLICHE MESSUNG VON VIBROAKUSTISCHEN EREIGNISSEN IN DER SYNCHRONISATION MIT EINEM SCHALLSIGNAL AUF EINER BERÜHRUNGSBILDSCHIRMVORRICHTUNG

HIGH-PRECISION TEMPORAL MEASUREMENT OF VIBRO-ACOUSTIC EVENTS IN SYNCHRONISATION WITH A SOUND SIGNAL ON A TOUCH-SCREEN DEVICE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **21.12.2018   FR 1873999**

(43) Date de publication de la demande:
**27.10.2021   Bulletin 2021/43**

(73) Titulaires:
• **UNIVERSITE DE MONTPELLIER
34090 Montpellier (FR)**
• **Naturalpad
34730 Prades-Le-Lez (FR)**

(72) Inventeurs:
• **DALLA BELLA, Simone
Montréal, Québec H4V 2B5 (CA)**
• **ANDARY, Sébastien
34980 SAINT-GELY DU FESC (FR)**

(74) Mandataire: **Gevers & Orès
Immeuble le Palatin 2
3 Cours du Triangle
CS 80165
92939 Paris La Défense Cedex (FR)**

(56) Documents cités:
EP-A1- 3 454 339          WO-A1-2005/122898
US-A1- 2014 249 447      US-A1- 2016 110 021

**Description**

**Domaine technique de l'invention**

L'invention concerne un procédé de détermination des temps de tape d'un utilisateur en réponse à un stimulus auditif. Au fin de la mise en oeuvre du procédé selon l'invention, on dispose d'un dispositif à écran tactile qui sera mieux décrit dans la suite.

Dans le cadre de l'invention, l'utilisateur est une personne apte à percevoir au moins un signal sonore. Des références à certaines parties du corps de l'utilisateur, notamment ses doigts ou ses mains, n'ont vocation qu'à mieux comprendre les interactions de que l'utilisateur est susceptible d'avoir avec le dispositif à écran tactile utilisé pour la mise en oeuvre du procédé de l'invention.

Le procédé selon l'invention trouve notamment son application dans l'étude de la synchronisation auditivo-motrice. Le procédé selon l'invention pourrait être mis en oeuvre dans le cadre d'études expérimentales cherchant à analyser la coordination auditivo-motrice chez l'utilisateur, ou simplement pourrait être mis en oeuvre dans des jeux éducatifs visant à améliorer la coordination auditivo-motrice chez l'enfant par exemple. Il s'agit d'exemples d'applications non limitatifs de la présente invention.

**Arrière-plan technique**

On connait les méthodes de coordination visuo-motrices dont le but est d'étudier la coordination des mouvements de certains segments du corps d'une personne, avec l'information visuelle perçue simultanément par ladite personne. Récemment, de telles méthodes ont été déployées sur des dispositifs à écran tactile en vue de mesurer le temps de réaction d'un utilisateur en réponse à des stimuli visuels émis depuis un dispositif à écran tactile.

On peut citer le document US 2014/0249447 qui décrit une méthode reposant sur l'enregistrement des vibrations acoustiques générées par un utilisateur lorsqu'il tapote sur un dispositif à écran tactile en réponse à des stimuli visuels, constituant des cibles pour l'utilisateur.

Le principe de la méthode est le suivant. Une série d'instructions est initialisée au moyen d'un programme installé sur un dispositif de type tablette. Le programme demande à la tablette de débuter l'enregistrement des vibrations acoustiques. Cet enregistrement étant stocké dans un fichier audio sur un support approprié de la tablette de même que la chronologie dudit enregistrement. L'utilisateur peut alors démarrer un essai en tapotant sur le centre d'un l'écran tactile du dispositif. Ensuite, le programme demande à la tablette d'éclairer l'une des cibles qui s'affichent sur l'écran et d'écrire l'heure à laquelle la cible s'est éclairée dans le journal. Le but est que l'utilisateur touche alors la cible qui s'est éclairée. Concomitamment, le microphone de la tablette permet de capter les vibrations créées par le contact de l'utilisateur avec la cible et l'heure à laquelle les vibrations sont détectées par ledit microphone est indiquée dans le journal. La tablette reçoit alors une notification de contact de l'écran tactile et les coordonnées précises du lieu du contact et écrit l'heure à laquelle la notification a été reçue et les coordonnées de la zone, sur l'écran tactile, où s'est produit le contact.

Dans la méthode décrite, la synchronisation des stimuli visuels avec les tapes de l'utilisateur est rendue possible en stockant la chronologie des événements issus de vibrations acoustiques et de l'éclairage des cibles dans le système de fichiers journaux de la tablette. Le temps de tape n'est pas déterminé à l'issu de cette synchronisation. Cette méthode de synchronisation n'est pas applicable à un procédé faisant appel à des stimuli auditifs puisque dans ce cas il n'est pas possible de stocker précisément la chronologie du stimulus auditif lu par des haut-parleurs dans les fichiers journaux, et encore moins pour un son émis par un dispositif émetteur extérieur à la tablette.

On connait également les méthodes dans lesquelles des traitements sont appliqués à un signal audio enregistré par le microphone d'un dispositif à écran tactile ou par des capteurs placés à proximité de l'écran tactile du dispositif. Dans la plupart des cas, on cherche à supprimer des vibrations indésirables générées par les tapes d'un utilisateur sur le dispositif à écran tactile ou tout autre évènement tel qu'un contact involontaire avec le dispositif, sans extraire d'information à partir des pics du signal associés à ces vibrations.

Le document EP 2 875 419 divulgue une telle méthode. Le principe de la méthode est le suivant. Pour un ou plusieurs capteurs, un signal audio émis par un microphone est traité pour déterminer une ou plusieurs parties du signal associées à une touche de l'écran tactile. Une estimation conjointe d'une pluralité des parties déterminées est effectuée pour isoler un signal caractéristique d'une frappe de l'utilisateur sur l'écran tactile. La partie identifiée peut inclure une composante de signal souhaitée, telle qu'une voix humaine enregistrée, et une composante de signal indésirable, tel que le son associé aux frappes de l'utilisateur sur l'écran tactile. Le signal caractéristique d'une frappe sur l'écran tactile déterminé à l'étape précédente est supprimé du signal audio correspondant pour chacun des signaux audio reçus. Un combinateur de signal peut alors être utilisé pour faire correspondre l'emplacement temporel du signal caractéristique de la frappe avec la partie correspondante dans le signal reçu lorsqu'il est soustrait dudit signal. Les signaux audio résultants sont alors additionnés et délivrés.

Le document WO 2005/122898 A1 divulgue une méthode de détermination d'un temps de réaction ou d'un temps de latence, le méthode comprenant : un moyen de commande commandant la fourniture d'un premier signal, qui peut être un signal sonore, à un utilisateur, un moyen de réception recevant une réaction de l'utilisateur, déterminant le temps de réaction ou le temps de latence comme une période de temps s'écoulant entre la fourniture du signal et la réception de la réaction, dans lequel : l'étape de fourniture comprend également la fourniture d'un second signal, qui peut également être un signal sonore, il y a une relation de synchronisation prédéterminée entre la fourniture des premier et second signaux, l'étape de réception comprend l'identification du second signal, et l'étape de réception comprend la détermination du temps de réaction ou du temps de latence sur la base de l'identification du second signal, de la réception de la réaction, et de la relation de synchronisation prédéterminée.

Les méthodes proposées jusqu'ici ne s'intéressent pas à la mesure temporelle à haute précision des temps de tapes d'un utilisateur en synchronisation avec un signal sonore sur un dispositif à écran tactile.

**Résumé de l'invention**

L'invention propose un procédé de détermination des temps de tape d'un utilisateur en réponse à un stimulus auditif, le procédé comprenant les étapes suivantes :

- lecture du stimulus auditif, ledit stimulus auditif correspondant au signal audio de référence,
- enregistrement du signal audio de référence et d'un ou plusieurs évènements vibro-acoustique, lesdits évènements vibro-acoustiques faisant suite à une ou plusieurs tapes de l'utilisateur sur un écran tactile d'un dispositif à écran tactile en réaction au signal audio de référence, le signal résultant de l'enregistrement étant appelé signal audio enregistré,
- détection du signal audio de référence dans le signal audio enregistré.

Le procédé est caractérisé en ce que :

- le signal audio enregistré est enregistré au moyen d'un microphone du dispositif à écran tactile,
- lors de l'étape de détection du signal audio de référence dans le signal audio enregistré, le signal audio de référence et le signal audio enregistré sont placés sur une échelle temporelle commune unique,
- il comprend une étape de filtrage dans laquelle le signal enregistré obtenu subséquemment à l'étape de détection, est préalablement filtré, puis normalisé de manière à conserver uniquement les fréquences correspondant aux évènements vibro-acoustiques générés par les actions de l'utilisateur sur l'écran tactile, et en ce que
- il comprend une étape de détection des événements vibro-acoustiques dans laquelle des instants associés auxdits évènements vibro-acoustiques sont identifiés dans le signal obtenu à la fin de l'étape de filtrage.

Le procédé selon l'invention permet ainsi d'une part d'identifier le début du signal audio de référence dans le signal audio enregistré et à partir de là de pouvoir déterminer les instants associés aux tapes de l'utilisateur sur l'écran tactile.

Selon différentes caractéristiques de l'invention qui pourront être prises ensemble ou séparément :

- l'étape de détection du signal audio de référence dans le signal audio enregistré comprend une première sous-étape de normalisation du signal audio enregistré de sorte à obtenir un signal audio enregistré normalisé ;
- l'étape de détection du signal audio de référence dans le signal audio enregistré comprend une deuxième sous-étape dans laquelle le signal audio de référence est ré-échantillonné au même taux que le signal audio enregistré et normalisé de sorte à obtenir un signal audio de référence ré-échantillonné et normalisé ;
- l'opération de normalisation réalisée lors de l'étape de détection du signal audio de référence dans le signal audio enregistré est effectuée au moyen d'une fonction de normalisation ;
- l'étape de détection du signal audio de référence dans le signal audio enregistré comprend, suite à la première et la deuxième sous-étape, une troisième sous-étape d'identification du début du signal audio de référence ré-échantillonné et normalisé dans le signal audio enregistré normalisé ;
- la troisième sous-étape comprend une sous-étape de construction d'un filtre adapté de sorte à identifier dans le signal audio enregistré normalisé l'instant d'échantillonnage le plus probable qui correspond au début des K premiers échantillons du signal audio de référence ré-échantillonné et normalisé,
- la troisième sous-étape comprend une sous-étape de détermination d'une fenêtre temporelle de taille dans laquelle effectuer la recherche du signal audio de référence ré-échantillonné normalisé dans le signal audio enregistré normalisé,
- la troisième sous-étape comprend une sous-étape de détermination de l'instant d'échantillonnage ;
- l'étape de filtrage comprend une première sous-étape de filtrage et optionnellement une deuxième sous-étape de filtrage ;

- la première sous-étape de filtrage est mise en oeuvre au moyen d'un filtre passe-bande de type Butterworth d'ordre 1 ayant une fréquence basse de 50 Hz et une fréquence haute de 200 Hz, et
- la seconde sous-étape de filtrage est mise en oeuvre au moyen d'un filtre passe-bas de type Butterworth d'ordre 1 ayant une fréquence haute de 400 Hz,
  de sorte qu'à la fin de la deuxième sous-étape de filtrage on obtient un signal audio enregistré normalisé filtré;
- suite à la deuxième sous-étape de filtrage, l'étape de filtrage comprend une troisième sous-étape de normalisation locale du signal audio enregistré normalisé filtré obtenu à la fin de la seconde sous-étape de filtrage de sorte à obtenir un signal audio enregistré normalisé filtré, ladite sous-étape de normalisation locale étant effectuée au moyen d'une fonction de normalisation locale ;
- suite à l'étape de filtrage on met en oeuvre l'étape de détection des évènements vibro-acoustiques ;
- l'étape de détection des évènements vibro-acoustiques comprend une première sous-étape de détermination de l'énergie du signal audio enregistré normalisé filtré, ladite première sous-étape étant effectuée au moyen d'une fonction énergie ;
- l'étape de détection des évènements vibro-acoustiques comprend, suite à la première sous-étape, une deuxième sous-étape de lissage du signal obtenu à la fin de la première sous-étape au moyen d'une fonction lissage définie par le produit de convolution du signal avec une fenêtre de pondération de type Hamming ;
- l'étape de détection des évènements vibro-acoustiques comprend, suite à la deuxième sous-étape, une troisième sous-étape d'extraction à partir du signal lissé de l'ensemble des P instants d'échantillonnage correspondant à des maxima locaux et/ou candidats de début d'évènements vibro-acoustiques ;
- l'étape de détection des évènements vibro-acoustiques comprend, suite à la troisième sous-étape, une quatrième sous-étape de présélection des candidats de début d'évènements vibro-acoustique ;
- la quatrième sous-étape comprend une sous-étape de regroupement de candidats associés aux P instants d'échantillonnage selon un premier critère de sélection, ledit premier critère de sélection correspondant au regroupement des instants d'échantillonnage candidats espacés par un nombre d'échantillons m prédéterminée, de sorte à former des groupes de maxima locaux ;
- la quatrième sous-étape comprend une sous-étape de conservation dans chaque groupe les instants associés aux maxima locaux pour lesquels la valeur maximale de l'énergie est obtenue ;
- l'étape de détection des évènements vibro-acoustiques comprend, suite à la quatrième sous-étape, une cinquième sous-étape d'élimination des maxima parasites ;
- la cinquième sous-étape comprend une sous-étape de tri, par hauteur décroissante, des maxima locaux conservés à la fin de la quatrième sous-étape ;
- la cinquième sous-étape comprend une sous-étape de conservation des $\rho N_{tap}$ plus grands maxima locaux, $N_{tap}$ étant le nombre d'évènements vibro-acoustiques compris dans la fenêtre temporelle de mesure et $\rho$ étant strictement supérieur à 1, $\rho > 1$ ;
- le procédé comprend en outre, suite à l'étape de détection des évènements vibro-acoustique, une étape supplémentaire d'optimisation du signal obtenu à la fin de ladite étape de détection des évènements vibro-acoustiques,
- ladite étape d'optimisation comprend une sous-étape d'appariement de l'ensemble des instants $t_i$, avec $i < \rho N_{tap}$, des maxima locaux conservés à l'étape de conservation des $\rho N_{tap}$ plus grands maxima locaux avec l'ensemble des instants modèles $t_j$, avec $j < N_{tap}$, mesurés par l'écran tactile ;
- ladite étape d'optimisation comprend une sous-étape d'évaluation de la qualité de l'appariement effectué lors de la sous-étape 5600) au moyen d'une fonction objectif,

  - ladite étape d'optimisation comprend une sous-étape de maximisation de la fonction objectif au moyen d'un paramètre,
  - ladite étape d'optimisation comprend une sous-étape de sélection des maxima locaux associés aux instants d'échantillonnage qui sont appariés exactement aux instants modèles mesurés par l'écran tactile ;

- l'étape d'optimisation comprend, suite à la quatrième sous-étape, une cinquième sous-étape d'ajustement dans laquelle les maxima locaux sélectionnés à l'issu de l'étape quatrième sous-étape sont ajustés de sorte à se conformer au signal audio enregistré ;
- l'ajustement est effectué en appliquant une fonction de compensation de déphasage aux maxima.

L'invention concerne également un dispositif à écran tactile adapté pour la mise en oeuvre du procédé tel que décrit précédemment. Le dispositif comprend un écran tactile, un microphone, une unité centrale de traitement configurée pour exécuter au moins les étapes du procédé tel que décrit précédemment.

L'invention concerne en outre un programme d'ordinateur comprenant des instructions qui lorsque le programme est exécuté par l'ordinateur conduisent celui-ci à mettre en oeuvre le procédé tel que décrit précédemment.

L'invention concerne également un moyen de stockage pour ordinateur sur lequel est enregistré le programme d'ordinateur tel que décrit précédemment.

## Brève description des figures

D'autres objets, caractéristiques et avantages de l'invention apparaîtront plus clairement dans la description qui suit, faite en référence aux figures annexées, dans lesquelles :

- la figure 1 illustre de manière schématique le processus d'acquisition du signal issu des tapes de l'utilisateur,

- la figure 2 illustre de manière schématique le traitement de l'enregistrement et l'extraction des instants associés aux tapes de l'utilisateur et du référentiel temporel,

- la figure 3 illustre les étapes du procédé de détermination des temps de tape de l'utilisateur en réponse à un stimulus auditif,

- la figure 4 illustre les sous-étapes de l'étape de détection du signal audio de référence dans le signal audio enregistré, les sous-étapes secondaires sont illustrées en pointillés,

- la figure 5 illustre les sous-étapes de l'étape de filtrage du signal enregistré obtenu subséquemment à la troisième étape et de normalisation,

- la figure 6 illustre les sous-étapes de l'étape de détection des évènements vibro-acoustiques dans le signal obtenu à la fin de la quatrième étape,

- la figure 7 illustre les sous-étapes de l'étape de détection des évènements vibro-acoustiques dans le signal obtenu à la fin de la quatrième étape (suite),

- la figure 8 illustre les sous-étapes de l'étape d'optimisation du signal obtenu à la fin de la cinquième étape.

## Description détaillée de l'invention

### A. Commentaires généraux et terminologie

Les termes définis dans la présente section sont applicables à l'ensemble des modes de réalisation divulgués dans la description qui suit.

Dans le cadre de l'invention, un dispositif à écran tactile comprend au moins un écran tactile, un système audio comportant au moins un microphone et un haut-parleurs, un programme de lecture et d'enregistrement audio, un programme d'ordinateur apte à mettre en oeuvre le procédé selon l'invention.

Le dispositif à écran tactile comprend en outre au moins une unité de traitement qui peut être définit par une unité de calcul de type processeur (*Central Processing Unit, CPU en anglais*), ledit processeur étant apte à exécuter le programme de lecture et d'enregistrement audio et le programme d'ordinateur apte à mettre en oeuvre le procédé selon l'invention.

Suivant l'architecture, le dispositif à écran tactile comprend en outre au moins une unité de calcul de type processeur graphique (*Graphies Processing Unit, GPU en anglais*), dédiée au calcul matriciel parallélisable, disposant d'une mémoire vive (*Random Access Memory, RAM en anglais*) et d'un moyen de stockage, par exemple une mémoire vive (*Random Access Memory, RAM en anglais*), une mémoire morte effaçable et programmable électriquement ou mémoire flash (*Electrically-Erasable Programmable Read-Only Memory, EEPROM en anglais*). Le programme de lecture et d'enregistrement audio est enregistré sur ledit moyen de stockage. De même, le programme apte à mettre en oeuvre le procédé selon l'invention est enregistré sur le moyen de stockage.

Le procédé peut également être implémenté au moyen d'un processeur ASIC (*application-specific integrated circuit en anglais*) ou encore un réseau de portes programmables *in situ* (*Field-programmable gate array, FPGA en anglais*) ou directement implémenté au moyen d'un microprocesseur optimisé pour exécuter des applications de traitement numérique du signal (*Digital Signal Processor, DSP en anglais*). Ces types de processeurs permettent ainsi d'améliorer les performances de traitement dans le dispositif à écran tactile.

De préférence, le dispositif à écran tactile est un dispositif mobile tel qu'une tablette tactile ou un smartphone. De manière plus général, le dispositif à écran tactile peut être un ordinateur équipé d'un écran tactile.

La structure du dispositif à écran tactile ou encore bâti sert de support à tous les autres éléments du dispositif, nommément l'écran tactile, le système audio, le processeur.

L'écran tactile consiste en un écran interactif permettant d'acquérir des données lors d'évènements affectant sa surface. La surface est plus précisément une dalle interactive ou surface d'interaction, de préférence, plane et de forme rectangulaire.

Dans le cadre de l'invention, la surface d'interaction est apte à subir des déformations instantanées, c'est-à-dire apte à vibrer et à dissiper l'énergie vibratoire créée lors de contacts avec ladite surface d'interaction. Le(s) contact(s) peut(vent) résulter soit d'une interaction entre les doigts de l'utilisateur et la surface d'interaction ou d'une interaction entre un stylet prévu à cet effet et ladite surface d'interaction ou de tout autre objet avec la surface d'interaction. En tout état de cause, le contact a nécessairement pour origine une action de l'utilisateur. Dans le cadre de l'invention, cette action est une frappe, une tape ou une touche de l'utilisateur.

L'énergie vibratoire émise par la surface d'interaction lors des tapes est apte à se propager, de proche en proche, à travers les éléments du dispositif à écran tactile sous forme d'onde en induisant une déformation élastique des différents éléments du dispositif traversés par l'onde à l'origine d'ondes acoustiques. Un « chemin vibratoire » est créé entre la surface d'interaction et les éléments du dispositif traversés par ladite onde. En particulier, l'énergie vibratoire dissipée par la surface d'interaction est, a *minima,* apte à se propager depuis ladite surface d'interaction jusqu'au microphone, par le biais de la structure du dispositif ou de tout autre élément du dispositif, et apte à interférer avec ledit microphone. Dans le cadre de l'invention, on qualifiera un tel phénomène de signal vibro-acoustique. En outre, on désignera le contact avec la surface d'interaction à l'origine du signal vibro-acoustique par le terme d'évènement vibro-acoustique, noté $e_{va}$.

L'écran tactile peut en outre comprendre un système de détection. Le système de détection définit le type d'écran tactile utilisé. Il est composé d'une pluralité d'éléments de détection permettant d'identifier les coordonnées des zones où se produisent des contacts avec l'écran tactile. Il existe différentes modes de détection dans les écrans tactiles. Pour les premiers, un contact direct avec l'écran tactile est nécessaire pour déclencher la détection, tandis que pour les seconds, il n'est pas nécessaire qu'il y ait un toucher de l'écran tactile pour que la détection puisse avoir lieu.

Les éléments de détection sont classiquement des capteurs, des détecteurs ou des émetteurs. Ils peuvent être localisés sous la surface d'interaction ou dans des coins de chaque côté de l'écran. Ces éléments de détection permettent d'obtenir la mesure du nombre de frappes de l'utilisateur par l'écran tactile, qui est une donnée d'entrée dans le cadre de l'invention. Quoi qu'il en soit, l'écran tactile utilisé pour mettre en oeuvre l'invention n'est pas limité à un mode de détection particulier.

Le haut-parleur peut être tout appareil apte produire ou émettre un(des) son(s) à partir d'un(de) signal(aux) électrique(s). En d'autres termes, le haut-parleur est tout appareil capable de convertir un signal électrique en un(des) son(s). Ces sons peuvent notamment provenir d'une musique ou encore d'un audio préenregistré dans la mémoire du dispositif et dont la lecture par le(s) haut-parleur(s) est apte à être déclenchée par l'utilisateur par le biais du programme de lecture et d'enregistrement audio. Ils sont détectables par le microphone du dispositif. De plus, l'application de l'invention à l'étude de la synchronisation auditivo-motrice requière, dans le cas où il est fait appel à un haut-parleur, que ces sons puissent être perceptibles par l'utilisateur du dispositif à écran tactile. Cela dit, il n'est pas obligatoire d'utiliser le(s) haut-parleur(s).

Le(s) son(s) peut(vent) être émis par un métronome. De manière similaire à un(des) son(s) qui serai(en)t émis par le haut-parleur, l'application de l'invention à la synchronisation auditivo-motrice requière que le son émis par un tel instrument puisse être au moins perceptible par l'utilisateur du dispositif à écran tactile. Le métronome pourra éventuellement être utilisé en combinaison avec le(s) haut-parleur(s). D'autres dispositifs connus de l'homme du métier pour émettre des sons, par exemple des instruments de musique, peuvent être utilisés seuls ou en combinaison selon l'application envisagée. L'invention n'est pas limitée aux exemples précités.

Les sons émis par le(s) haut-parleur(s) et/ou le métronome et/ou le dispositif émetteur de son sont appelés stimuli auditifs. Dans le cadre de l'invention, les stimuli auditifs générés par le dispositif émetteur de son sont appelés signal audio de référence ou signal de référence, noté $S_R$. En réponse à ces stimuli auditifs, et selon leur durée, l'utilisateur est susceptible d'avoir une ou plusieurs réactions, ici, il est notamment amener à toucher la surface d'interaction de l'écran tactile, et amener à provoquer un ou plusieurs évènements vibro-acoustiques, $e_{va}$.

Les sons émis par le(s) haut-parleur(s) et/ou le métronome peuvent être précédés et/ou entrecoupés d'un(de) silence(s). On entend par silence, une absence de son. Autrement dit, le silence correspond à une interruption de son.

Le microphone peut être tout appareil permettant de détecter un(des) signal(aux) acoustique(s) et/ou vibro-acoustique(s) et apte à convertir ledit(lesdits) signal(aux) en signal(aux) électrique(s). Le microphone est capable d'enregistrer un(des) signal(aux) acoustique(s). Il permet de fournir une tension électrique proportionnelle aux variations acoustiques. Le signal acoustique peut avoir pour origine un(des) évènement(s) vibro-acoustique(s) sur la surface d'interaction, $e_{va}$, un(des) son(s) provenant des bruits dans l'environnement immédiat du dispositif, par exemple le son émis par une voix humaine, l'émission de sons par le(es) haut-parleur(s) du dispositif. Le signal acoustique peut encore venir d'un métronome comme mentionné précédemment. Le signal acoustique peut subir des dégradations dues à d'éventuels bruits venant de l'appareillage lui-même.

Les signaux acoustiques d'intérêt visent en particulier les sons émis par le dispositif émetteur de sons (éventuellement précédés et/ou entrecoupés de silences) et ceux résultant des tapes de l'utilisateur (évènements vibro-acoustiques, également éventuellement précédés et/ou entrecoupés de silences).

Les signaux vibro-acoustique, $S_{va}$, résultants des évènements $e_{va}$ se situent dans une gamme de fréquences particulièrement basses, bien en deçà des ondes sonores. La précision de détection et d'enregistrement de tels signaux par le microphone est uniquement limitée par les capacités du microphone lui-même. Classiquement, pour les dispositifs de type tablettes tactiles et smartphones, les microphones utilisés présentent une fréquence d'échantillonnage comprise entre 8 et 48 kHz. Ces valeurs ne sont pas limitatives dans le cadre de la présente invention. Bien qu'une précision de détection optimale soit préférable, elle n'est pas nécessaire à la mise en oeuvre du procédé selon l'invention

La précision de détection est au sens large la fidélité de reproduction par le microphone du(es) son(s) émis par le dispositif les haut-parleurs et/ou le métronome et/ou le dispositif émetteur de son. La précision de détection ne doit pas être confondue avec la qualité de l'enregistrement ou du signal enregistré. La qualité du signal audio enregistré dépend, en plus de la précision de détection, de la nature, de la forme, etc. de l'objet qui heurte la surface d'interaction mais aussi de la vitesse avec laquelle cet objet heurte la surface. À titre d'exemple, les vibrations mécaniques et/ou sons émis par un stylet seront perçus plus distinctement que les vibrations mécaniques et/ou sons émis par un doigt de l'utilisateur lorsque celui-ci porte des gants, indépendamment du mode de détection de l'écran tactile.

Dans les sections qui suivent, on s'intéresse plus précisément au traitement du signal et/ou des signaux.

Le signal de référence $S_R$ et les vibrations mécaniques issues des tapes de l'utilisateur sont enregistrés par le microphone. Le signal audio de référence $S_R$ et les vibrations mécaniques sont susceptibles d'interagir et de créer des interférences. Ces interférences sont à l'origine d'un signal résultant complexe formé à partir de la superposition de vibrations simples. Ces vibrations simples peuvent avoir des amplitudes et des fréquences différentes et déterminées de sorte que le signal résultant peut subir des variations de fréquence et/ou d'amplitude dans le temps. Le signal résultant peut être de forme quelconque. Il peut être formé d'un mélange de signaux sinusoïdaux. Ce signal résultant est transmis au microphone puis enregistré par ce dernier.

Le signal transmis et enregistré par le microphone est appelé signal audio enregistré ou signal enregistré, noté $S_E$. Comme mentionné précédemment, le(s) signal(aux) acoustique(s) et/ou vibro-acoustique(s) détecté(s) par le microphone est(sont) transformé(s) en signal(aux) électrique(s), lui(eux)-même(s) converti(s) et enregistré(s) sous forme de données numériques à la fréquence d'échantillonnage du dispositif à écran tactile utilisé, dans la mémoire dudit dispositif. À ce propos, l'opération durant laquelle le signal résultant est prélevé ou encore mesuré à intervalles définis, notamment selon le taux d'échantillonnage du microphone, et réguliers est appelé ici échantillonnage. Le microphone pourra, également et de préférence, être associé à un dispositif de conversion présent dans le dispositif à écran tactile.

Dans le cadre de l'invention, on cherche à synchroniser le début du signal de référence $S_R$ et les évènements vibro-acoustiques $e_{va}$ afin de déterminer le nombre de tapes de l'utilisateur. Or, l'instant de début d'émission du signal de référence $S_R$ relativement à celui auxquels débutent les évènements vibro-acoustiques $e_{va}$ est inconnu puisque l'horloge du processeur est lui-même inconnu. À cet égard, le signal de référence $S_R$ et le signal enregistré $S_E$ sont donc susceptibles de subir des transformations mathématiques successives, appelées aussi opérations de traitement du signal ou traitements et décrits dans la suite.

L'opération de ré-échantillonnage consiste à porter un premier signal et un deuxième signal à la même fréquence d'échantillonnage et à la même amplitude. Pour cela, l'un desdits premier et deuxième signaux est normalisé, par exemple le premier signal. L'autre signal, c'est-à-dire le deuxième signal, subit un ré-échantillonnage à la même fréquence, i.e. au même taux d'échantillonnage, que le premier signal, puis il est normalisé également. En d'autres termes, le deuxième signal est « réenregistré » dans un format d'échantillonnage différent de celui qu'il avait initialement. Par exemple, le premier signal peut être le signal enregistré $S_E$ tandis que le deuxième signal peut être le signal de référence $S_R$.

La normalisation s'entend ici comme l'opération consistant à modifier et/ou corriger l'amplitude d'un signal paramètre de sorte que, sur une fenêtre temporelle de mesure, la valeur maximale de l'amplitude dudit signal paramètre atteigne une valeur prédéterminée, et que le correctif apporté pour cette valeur maximale de l'amplitude soit également appliquée au signal paramètre en entier, c'est-à-dire sur toute la fenêtre temporelle de mesure. Par exemple, le signal paramètre peut être le signal enregistré $S_E$.

Selon un premier mode de calcul, une fonction de normalisation peut être définie de la manière suivante :

$$f_{n1}(X) := \frac{X - moyenne\,(X)}{médiane\,(abs(X))} \tag{1}$$

Où

$X$ est le signal paramètre,
*moyenne (X)* est la valeur moyenne du signal X,

7

*abs (X)* est le signal dont les échantillons sont des valeurs absolues des échantillons ou signaux élémentaires du signal X,

*médiane (abs(X))* est la valeur médiane du signal abs (X).

La valeur moyenne, *moyenne (X)*, où valeur efficace correspond à l'amplitude moyenne du signal paramètre sur l'ensemble d'une fenêtre temporelle donnée. Autrement dit, il s'agit de l'amplitude moyenne de l'ensemble des échantillons ou signaux élémentaires qui composent le signal paramètre X.

La valeur médiane, *médiane (X)*, correspond à l'amplitude permettant de couper les amplitudes de l'ensemble des échantillons ou signaux élémentaires composant le signal paramètre X en deux parties comportant chacune le même nombre d'échantillons de sorte qu'une première moitié des échantillons ait des amplitudes supérieures à la valeur médiane et l'autre moitié des échantillons ait des amplitudes inférieures à ladite valeur médiane.

Le calcul de la fonction de normalisation est, ici, effectué dans la fenêtre de mesure, c'est-à-dire sur la totalité du temps d'enregistrement, appelé encore session. Autrement dit, la fonction de normalisation est appliquée à chacun des échantillons ou signaux élémentaires composant le signal paramètre X. Cela étant, la fenêtre temporelle utilisée peut avoir une taille plus limitée de sorte que le calcul de la fonction de normalisation soit réalisé dans une fenêtre temporelle limitée, dite fenêtre de pondération.

Dans un cas comme dans l'autre, le calcul de la fonction de normalisation est effectué dans une fenêtre temporelle « fixe » signifiant que la moyenne est calculée sur l'ensemble de la fenêtre temporelle considérée, i.e. la fenêtre de mesure ou la fenêtre de pondération. Autrement dit, la moyenne est calculée pour tous les échantillons compris dans la fenêtre temporelle considérée.

Le calcul de la fonction de normalisation peut également être réalisé dans une fenêtre temporelle, dite fenêtre glissante. La fenêtre temporelle glissante permet une transformation par moyenne mobile pondérée d'une grandeur associée au signal paramètre X pour chaque sous ensemble de la fenêtre de glissante et pour un nombre d'échantillons présélectionné. En l'espèce, dans un mode de calcul de la fonction de normalisation sur une fenêtre glissante, ladite fonction de normalisation est recalculée pour chaque sous-ensemble de la fenêtre glissante en vue d'obtenir les valeurs normalisées pour l'ensemble de ladite fenêtre glissante. Cela permet d'effectuer un lissage des valeurs en supprimant les fluctuations transitoires qui pourraient apparaître dans le signal. On parle alors ici d'une fonction de normalisation locale.

Selon un second mode de calcul, la fonction de normalisation locale peut être définie de la manière suivante :

$$f_{nloc}(X) := \frac{X - moyenne_{loc}(X)}{moyenne_{loc}(abs_{loc}(X - moyenne_{loc}(X)))} \qquad (2)$$

Où

*X* est le signal paramètre,

*moyenne$_{loc}$ (X)* est la valeur moyenne locale du signal X,

*abs$_{loc}$ (X - moyenne$_{loc}$ (X))* est le signal dont les échantillons sont des valeurs absolues locales des échantillons ou signaux élémentaires du signal X sur le sous-ensemble de la fenêtre glissante.

Les notions de « fenêtre de mesure », « fenêtre de pondération », « fenêtre fixe » et de « fenêtre glissante » peuvent être étendues aux différents traitements du signal et transformations mathématiques appliquées au signal paramètre de la présente invention.

En outre, une ou plusieurs opérations de filtrage peuvent également être appliquées au signal. L'opération de filtrage peut avoir plusieurs objectifs en fonction du traitement à effectuer.

On pourra construire et utiliser un filtre adapté pour localiser dans un premier signal paramètre, noté $X_1$, l'instant d'échantillonnage le plus probable qui correspond au début des K premiers échantillons d'un deuxième signal paramètre, noté $X_2$. Le filtre adapté fournit une amplification des échantillons du deuxième signal paramètre $X_2$ par rapport au premier signal paramètre $X_1$, et permet d'identifier la localisation de ces échantillons avec plus ou moins de précisions selon la méthode choisie pour construire ledit filtre. En d'autres termes, le filtre adapté permet de détecter temporellement et de maximiser un ou plusieurs échantillons dans ledit premier signal paramètre.

Une fenêtre temporelle de taille $T_2$ peut être définie pour restreindre la recherche du deuxième signal paramètre $X_2$ dans le premier signal paramètre $X_1$.

Dans un mode de réalisation de l'invention, l'instant d'échantillonnage $t_2$ correspondant au début du deuxième signal $X_2$ dans le premier signal $X_1$ est défini de la manière suivante :

$$t_2 := argmax_{t<T_2} \sum_{k=t}^{t+K} X_1(k) \cdot X_2(k-t) \qquad (3)$$

Où

> $t$ correspond à un instant d'échantillonnage donné,
> $t_2$ exprime l'instant d'échantillonnage correspondant au début du deuxième signal paramètre $X_2$ dans le premier signal paramètre $X_1$,
> $T_2$ est la taille de la fenêtre temporelle (en secondes).

L'argument maximum, noté **argmax**, désigne l'ensemble des échantillons, sur la fenêtre temporelle $T_2$, en lesquels le produit de convolution $\sum_{k=t}^{t+K} S_1(k) \cdot S_2(k-t)$ atteint sa valeur maximale.

Plus précisément, on cherche l'échantillon qui maximise le produit de convolution du premier signal paramètre $X_1$ par le deuxième signal paramètre $X_2$ renversé dans le temps. L'instant d'échantillonnage associé à cet échantillon constituera le temps de référence utilisé pour la synchronisation desdits premier et deuxième signaux paramètres $X_1$, $X_2$.

L'opération de synchronisation d'un premier signal paramètre $X_1$ avec un deuxième signal paramètre $X_2$ consiste à positionner lesdits signaux sur une échelle temporelle commune unique. Cette opération de synchronisation des signaux nécessite donc de pouvoir détecter avec précision le début du deuxième signal paramètre $X_2$ dans le premier signal paramètre $X_1$. L'instant d'échantillonnage associé au début du deuxième signal paramètre $X_2$ est alors considéré comme l'instant d'origine d'un référentiel temporel. En d'autres termes, cela consiste à repérer le temps d'échantillonnage qui correspond au début du signal audio de référence $S_R$. Ainsi, les moments des tapes et le moment du début du signal de référence $S_R$ sont positionnés sur une échelle temporelle commune unique.

D'autres méthodes pourront être envisagées par l'homme du métier pour construire le filtre adapté en tenant compte de la fréquence d'échantillonnage et de la taille de la fenêtre temporelle.

En outre, on pourra utiliser un filtre passe bande pour conserver dans un signal paramètre X uniquement les fréquences associées à des évènements utiles, par exemple les évènements vibro-acoustiques $e_{va}$, alors que les fréquences associées à tout autre évènement, par exemple un signal sonore, des bruits de l'environnement extérieur ou encore des bruits venant de l'appareillage lui-même, sont atténuées. Cette atténuation est possible car seuls les évènements ayant des fréquences localisées dans un intervalle de fréquences comprises entre une première fréquence limite, dite fréquence basse, et une deuxième fréquence limite, dite fréquence haute, du filtre ne sont conservées.

Dans un mode de réalisation de l'invention, le filtre passe-bande utilisé est un filtre de Butterworth. Le filtre de Butterworth est un filtre linéaire ayant, de manière avantageuse, une réponse relativement constante dans la bande passante.

L'invention n'est pas limitée à l'utilisation de filtres passe-bande de Butterworth. D'autres filtres passe-bande connus de l'homme du métier et ayant une réponse relativement constante dans la bande passante peuvent être envisagés, sans pour autant sortir du cadre de l'invention. A titre d'exemple, on peut citer le filtre de Bessel qui offre également une réponse très plate dans la bande passante.

En outre, en plus d'éventuelles opérations de normalisation et de filtrage, une ou plusieurs opérations de lissage peuvent être appliquées au signal paramètre X. L'opération de lissage permet d'atténuer des pics, dits pics secondaires, qui consisteraient à des perturbations ou bruits non associés à des évènements utiles, par exemple à des évènements vibro-acoustiques $e_{va}$ et de conserver uniquement les pics, dits pics majeurs, dudit signal paramètre X.

On entend par pic des valeurs du signal paramètre X correspondant à des maxima locaux. En principe, un pic secondaire présente une amplitude plus faible qu'un pic majeur.

Dans un mode de réalisation de l'invention, la fonction de lissage est définie par le produit de convolution du signal paramètre X avec une fenêtre de pondération de type Hamming de la façon qui suit :

$$\tilde{X} := X * Hamming\,(T_{lissage}) \qquad (4)$$

Où

> $\tilde{X}$ est le signal paramètre lissé,
> $X$ est le signal paramètre avant lissage,
> **Hamming** est la fenêtre de pondération,

$T_{lissage}$ est la taille de la fenêtre de pondération.

La fenêtre de pondération, Hamming, est une opération de transformation par moyenne mobile pondérée. Elle permet de lisser les valeurs du signal paramètre X en appliquant un plus grand coefficient d'observation lissée au centre de la fenêtre et des pondérations de plus en plus faibles à mesure que l'on s'éloigne du centre de la fenêtre.

La mise en oeuvre des opérations mentionnées ci-dessus, est rendue possible au moyen d'un programme dédié installé sur le dispositif à écran tactile. Le processeur permet d'exécuter les instructions reçues par ce programme et les programmes installés sur ledit dispositif à écran tactile.

## B. Description détaillée d'un mode de réalisation de l'invention

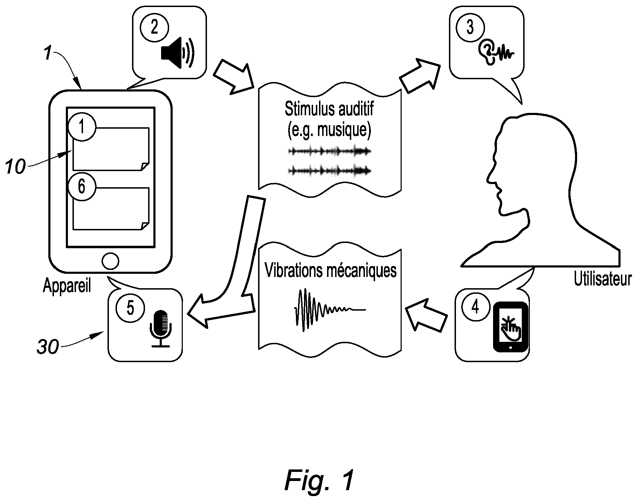

En référence à la figure 1, le processus d'acquisition du signal est décrit. Le programme de lecture et/ou enregistrement audio lance la lecture du stimulus auditif sur le dispositif à écran tactile 1.

Le stimulus auditif ou signal audio de référence $S_R$ est émis par les haut-parleurs du dispositif à écran tactile. L'utilisateur est alors apte à percevoir le signal audio de référence $S_R$, ce qui l'amène à toucher l'écran tactile 10 du dispositif et à générer un ou plusieurs évènements vibro-acoustique $e_{va}$ de manière synchronisée avec le signal audio de référence $S_R$.

Le microphone 30 du dispositif à écran tactile enregistre alors à la fois le signal audio de référence $S_R$ et les vibrations mécaniques générées par les évènements vibro-acoustiques générés par les tapes de l'utilisateur. Le programme enregistre alors le signal résultant, signal audio enregistré $S_E$, dans la mémoire du dispositif 1. La mémoire du dispositif peut être de type mémoire vive (*Random Access Memory, RAM en anglais*), mémoire morte effaçable et programmable électriquement ou mémoire flash (*Electrically-Erasable Programmable Read-Only Memory, EEPROM en anglais*).

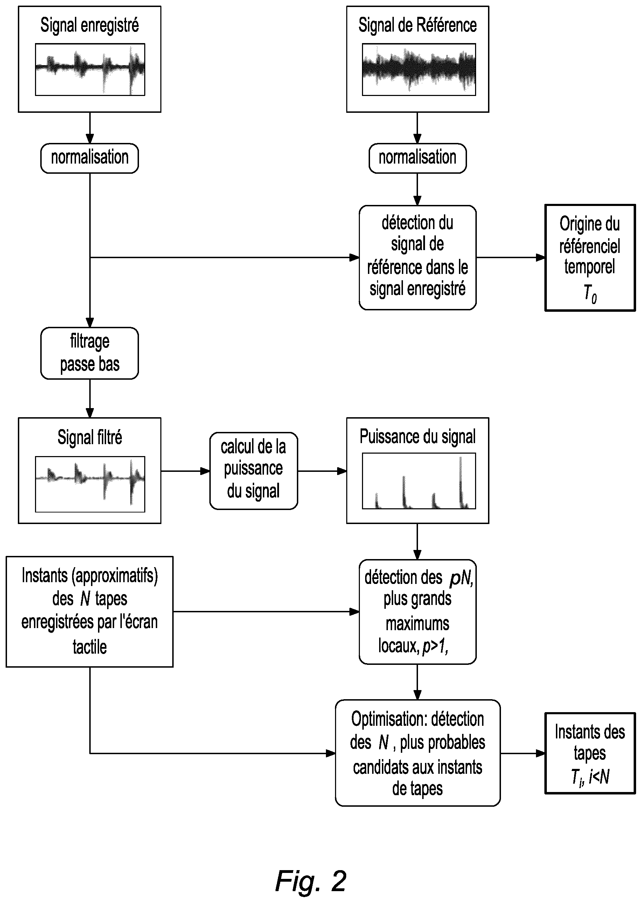

En référence à la figure 2, l'algorithme de traitement du signal audio enregistré $S_E$ et d'extraction des instants associés aux tapes de l'utilisateur et du référentiel temporel est illustré. Cette illustration permet de mieux comprendre la structure de l'algorithme de calcul.

Les entrées de l'algorithme sont le signal audio enregistré $S_E$, le signal audio de référence $S_R$ et le nombre de tapes mesurées par l'écran tactile 10 du dispositif. Les sorties de l'algorithme sont la localisation du début du signal audio de référence $S_R$ dans le signal audio enregistré $S_E$ et les instants des tapes de l'utilisateur.

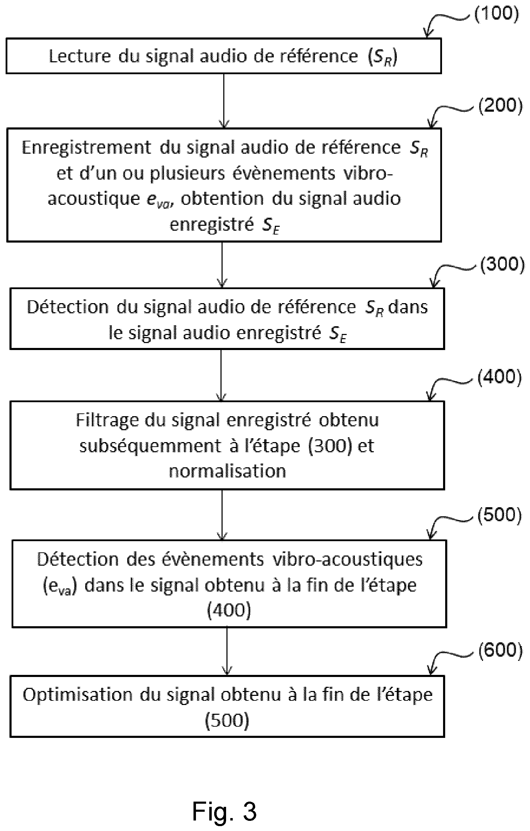

Comme illustré à la figure 3, le procédé de détermination des temps de tape de l'utilisateur selon l'invention comprend les étapes suivantes :

100) lecture d'un signal audio de référence $S_R$,

200) enregistrement du signal audio de référence $S_R$ et d'un ou plusieurs évènements vibro-acoustiques $e_{va}$, ledit enregistrement correspondant au signal audio enregistré $S_E$,

300) détection du signal audio de référence $S_R$ dans le signal audio enregistré $S_E$,

400) filtrage du signal enregistré obtenu lors de l'étape 300),

500) détection des évènements vibro-acoustique $e_{va}$ dans le signal obtenu à la fin de l'étape 400), et

600) optimisation du signal obtenu à la fin de l'étape 500).

Les étapes 100) à 600) sont mises en oeuvre dans l'ordre ci-dessus mentionné au moyen d'un programme d'ordinateur. Plusieurs étapes parmi les étapes 100) à 600) comprennent des sous-étapes. Ces sous-étapes ainsi que l'ordre dans lequel elles sont réalisées sont mieux décrits dans la suite. De manière générale, ces sous-étapes sont présentées dans l'ordre dans lequel elles sont effectuées.

La première étape 100) de lecture du signal audio de référence $S_R$ peut être réalisée au moyen d'un haut-parleur du dispositif 1 et/ou d'un métronome et/ou d'un dispositif émetteur de son, selon l'application visée. De préférence, on privilégie pour la lecture du signal audio de référence $S_R$ les haut-parleurs et/ou le métronome.

La deuxième étape 200) d'enregistrement est réalisée au moyen du microphone 30 du dispositif 1. L'enregistrement effectué lors de cette étape correspond au signal audio enregistré $S_E$. Le signal audio enregistré $S_E$ comprend une première composante principale issue du signal audio de référence $S_R$ et une seconde composante principale ayant pour origine les évènements vibro-acoustiques $e_{va}$. Ces composantes ne sont pas immédiatement différentiables et forment un signal audio enregistré complexe.

Comme mentionné précédemment, lesdits évènements vibro-acoustiques $e_{va}$ sont générés par les tapes de l'utilisateur sur l'écran tactile 10 du dispositif 1 en réaction au signal audio de référence $S_R$. Ces évènements se produisent donc de manière concomitante avec la lecture du signal audio de référence $S_R$. Plusieurs opérations de traitement du signal audio enregistré $S_E$ sont nécessaires pour pouvoir déterminer les instants et temps de ces évènements $e_{va}$ dans ledit signal audio de référence $S_R$. En effet, il est important de noter que les instants de détection $t_j$ mesurés par l'écran tactile ne suffisent pas car leur précision est comprise entre 50 et 300 millisecondes pour des dispositifs à écran tactile grand public vendus dans le commerce.

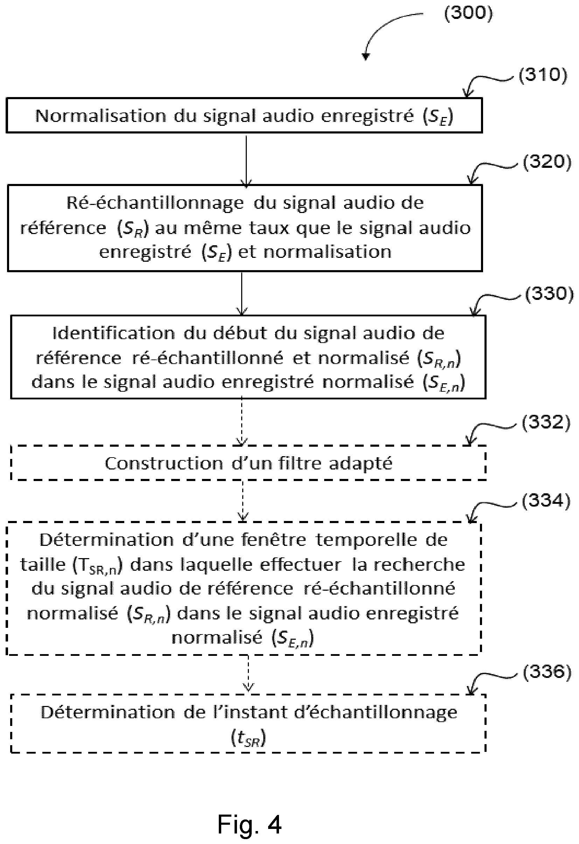

En référence à la figure 4, la troisième étape 300) de détection du signal audio de référence $S_R$ dans le signal audio enregistré $S_E$ vise à déterminer l'origine du référentiel temporel.

A cet égard, l'étape 300) peut comprendre une première sous-étape 310) de normalisation du signal audio enregistré $S_E$ au terme de laquelle un signal audio enregistré normalisé $S_{E,n}$ est obtenu.

L'étape 300) peut en outre comprendre une deuxième sous-étape 320) qui peut être mise en oeuvre concomitamment avec la première sous-étape 310). La deuxième sous-étape 320) consiste en un ré-échantillonnage du signal audio de référence $S_R$ au même taux que le signal audio enregistré $S_E$ et à une normalisation du signal de référence ré-échantillonné. Au terme de cette deuxième sous-étape 320), un signal audio de référence ré-échantillonné et normalisé $S_{R,n}$ est obtenu.

Avantageusement, les opérations de normalisation respectivement dudit signal audio enregistré $S_E$ et dudit signal audio de référence ré-échantillonné sont effectuées au moyen d'une fonction de normalisation définie par :

$$f_n(X) := \frac{X - moyenne\ (X)}{médiane\ (abs(X))} \tag{5}$$

Où

$X$ est ici le signal audio enregistré ($S_E$) ou le signal audio de référence ($S_R$) ré-échantillonné,
*moyenne (X)* est la valeur moyenne du signal considéré,
*abs (X)* est le signal dont les échantillons sont des valeurs absolues des échantillons du signal X et
*médiane (abs(X))* est la valeur médiane du signal abs (X).

La mise en oeuvre des première et deuxième sous-étapes 310) et 320) permet d'obtenir le signal de référence ré-échantillonné normalisé $S_{R,n}$ ayant la même fréquence d'échantillonnage et la même amplitude que le signal enregistré normalisé $S_{E,n}$. Ces sous-étapes permettent également de placer le signal de référence ré-échantillonné normalisé $S_{R,n}$ et le signal enregistré normalisé $S_{E,n}$ sur une échelle temporelle unique.

De préférence, l'étape 300) peut également comprendre, suite à la première sous-étape 310) et à la deuxième sous-étape 320), une troisième sous-étape 330) d'identification du début du signal audio de référence ré-échantillonné normalisé $S_{R,n}$ dans le signal audio enregistré normalisé $S_{E,n}$. La troisième sous-étape 330) comprend trois sous-étapes secondaires identifiées sur la fig. 4 par des encadrés en pointillés.

Une première sous-étape secondaire 332) est la construction d'un filtre adapté de sorte à identifier dans le signal audio enregistré normalisé $S_{E,n}$ l'instant d'échantillonnage $t_{SR}$ le plus probable qui correspond au début des K premiers échantillons du signal audio de référence ré-échantillonné et normalisé $S_{R,n}$. Le filtre adapté fournit une amplification des échantillons du signal audio de référence ré-échantillonné et normalisé $S_{R,n}$ par rapport au signal audio enregistré normalisé $S_{E,n}$, et permet d'identifier la localisation de ces échantillons avec plus ou moins de précisions selon la méthode choisie pour construire ledit filtre.

La troisième sous-étape 330) comprend en outre une deuxième sous-étape secondaire 334) de détermination d'une fenêtre temporelle de taille $T_{SR,n}$ dans laquelle effectuer la recherche du signal audio de référence ré-échantillonné normalisé $S_{R,n}$ dans le signal audio enregistré normalisé $S_{E,n}$. La taille $T_{SR,n}$ de la fenêtre temporelle correspond au nombre d'échantillons compris dans la fenêtre temporelle. L'utilisation d'une fenêtre temporelle de taille $T_{SR,n}$ permet de restreindre la recherche du début du signal audio de référence ré-échantillonné et normalisé $S_{R,n}$ dans le signal audio enregistré normalisé $S_{E,n}$.

La troisième sous-étape 330) comprend une troisième sous-étape secondaire 336) de détermination de l'instant d'échantillonnage $t_{SR}$. Cette étape vise en particulier à déterminer l'origine du référentiel temporel. La détermination de l'origine du référentiel temporel permet également de déterminer à partir de quel instant les événements vibro-acoustique $e_{va}$ sont susceptibles de se produire.

L'instant d'échantillonnage $t_{SR}$ est défini de la manière qui suit :

$$t_{S_R} := argmax_{t < T_{SR,n}} \sum_{k=t}^{t+K} S_{E,n}(k) \cdot S_{R,n}(k-t) \qquad (6)$$

Où

$t$ correspond à un instant d'échantillonnage donné,

$t_{SR}$ exprime l'instant d'échantillonnage correspondant au début signal audio de référence ré-échantillonné et normalisé $S_{R,n}$ dans le signal audio enregistré normalisé $S_{E,n}$,

$T_{SR,n}$ est la taille de la fenêtre temporelle en secondes, et

**argmax**, désigne l'ensemble des points, sur la fenêtre temporelle $TS_{R,n}$, en lesquels le produit de convolution

$\sum_{k=t}^{t+K} S_{E,n}(k) \cdot S_{R,n}(k-t)$ atteint sa valeur maximale.

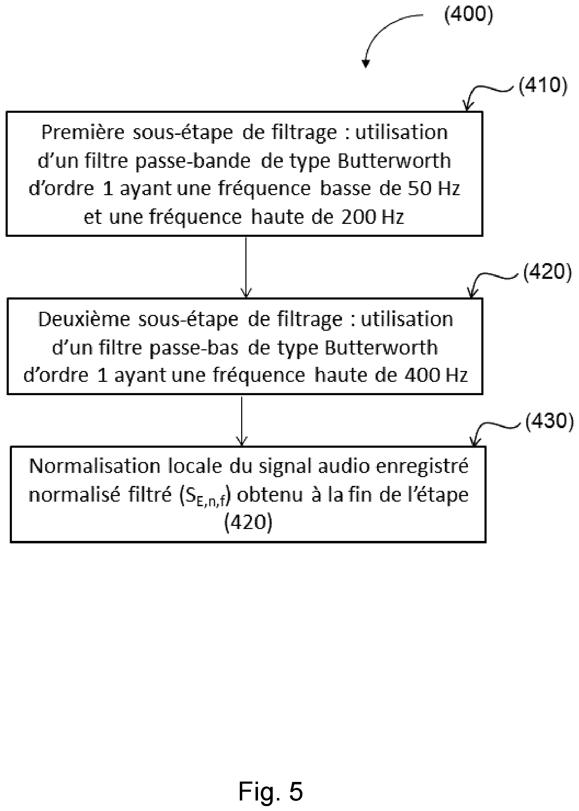

En référence à la figure 5, la quatrième étape 400) de filtrage du signal audio enregistré normalisé $S_{E,n}$ obtenu lors de l'étape 300) vise à conserver dans le signal audio enregistré normalisé uniquement les fréquences associées aux évènements vibro-acoustiques $e_{va}$. Concomitamment, les fréquences associées au signal de référence ré-échantillonné normalisé $S_{R,n}$ et celles associées aux éventuels bruits provenant de l'environnement extérieur ou encore des bruits venant de l'appareillage lui-même, sont atténuées.

A cet égard, l'étape 400) peut comprendre une première sous-étape 410) de filtrage du signal audio enregistré normalisé $S_{E,n}$. De préférence, elle est mise en oeuvre au moyen d'un filtre passe-bande de type Butterworth d'ordre 1 ayant une fréquence basse de 50 Hz et une fréquence haute de 200 Hz. Cela dit, la sous-étape 410) peut être mise en oeuvre au moyen de tout autre filtre passe-bande connu de l'homme du métier permettant d'atténuer les fréquences indésirables du signal audio enregistré normalisé $S_{E,n}$ tout en ayant une réponse relativement constante dans la bande passante.

L'étape 400) peut optionnellement comprendre une deuxième sous-étape 420) de filtrage du signal obtenu à l'issu de la première sous-étape 410) de filtrage. Elle est mise en oeuvre au moyen d'un filtre passe-bas de type Butterworth d'ordre 1 ayant une fréquence haute de 400 Hz. Elle permet d'atténuer encore plus les fréquences associées au signal de référence ré-échantillonné normalisé $S_{R,n,}$ et de manière générale toutes les composantes du signal audio enregistré normalisé $S_{E,n}$ qui ne sont pas associés aux tapes de l'utilisateur sur l'écran tactile 10. Un filtre de Bessel qui offre également une réponse très plate dans la bande passante peut également être utilisé.

Au terme de la première sous-étape 410) et, le cas échéant, de la deuxième sous-étape 420), un signal audio enregistré normalisé filtré $S_{E,n,f}$ est obtenu.

L'étape 400) comprend également une troisième sous-étape 430) de normalisation locale du signal audio enregistré normalisé filtré $S_{E,n,f}$ obtenu à la fin de l'étape 420). De préférence, la troisième sous-étape 430) est mise en oeuvre suite à la deuxième sous-étape 420).

L'opération de normalisation locale 430) est effectuée au moyen d'une fonction de normalisation locale définie par :

$$f_{nloc}(S_{E,n,f}) := \frac{S_{E,n,f} - moyenne_{loc}(S_{E,n,f})}{moyenne_{loc}(abs_{loc}(S_{E,n,f} - moyenne_{loc}(S_{E,n,f})))} \qquad (7)$$

Où

$S_{E,n,f}$ est le signal audio enregistré normalisé filtré,

**moyenne$_{loc}$ ($S_{E,n,f}$)** est la valeur moyenne locale du signal audio enregistré normalisé filtré $S_{E,n,f}$,

**abs$_{loc}$ ($S_{E,n,f}$ - moyenne$_{loc}$ ($S_{E,n,f}$))** est le signal dont les échantillons sont des valeurs absolues locales des échantillons du signal audio enregistré normalisé filtré $S_{E,n,f}$ sur le sous-ensemble d'une fenêtre glissante de taille $2T_{norm}$.

Au terme de la troisième sous-étape 430), un signal audio enregistré normalisé filtré $\widehat{S_{E,n,f}}$ est obtenu, un traitement de normalisation locale ayant été appliqué à ce signal.

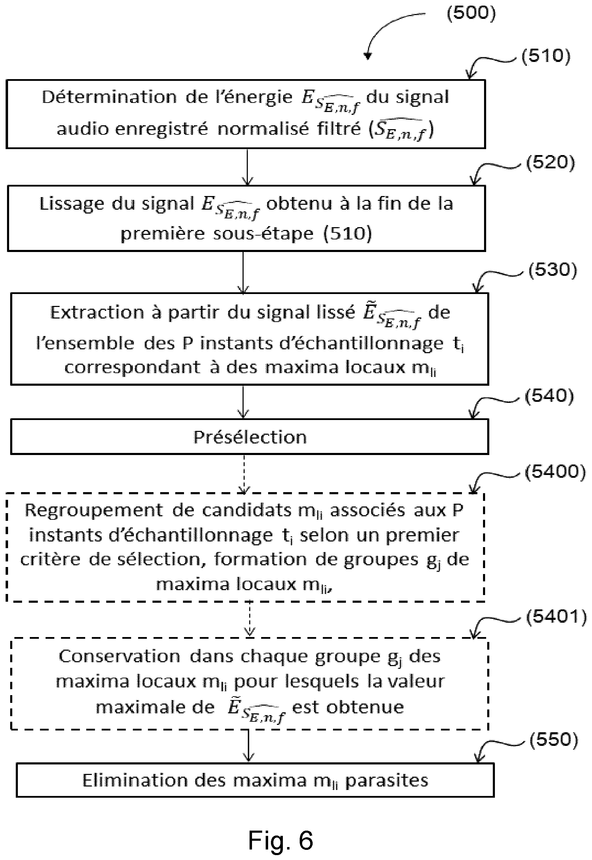

En référence à la figure 6, la cinquième étape 500) de détection des évènements vibro-acoustiques $e_{va}$ dans

le signal audio enregistré normalisé filtré $\widehat{S_{E,n,f}}$ vise à identifier dans ledit signal audio enregistré normalisé filtré $\widehat{S_{E,n,f}}$ les instants des pics associés aux évènements vibro-acoustiques $e_{va}$ générés par les tapes de l'utilisateur.

On entends par « pics », les échantillons présentant sur une fenêtre temporelle donnée des amplitudes maximales.

A cet égard, l'étape 500) comprend avantageusement une première sous-étape 510) de détermination de l'énergie $E_{\widehat{S_{E,n,f}}}$ du signal audio enregistré normalisé filtré $\widehat{S_{E,n,f}}$. De préférence, la première sous-étape 510) est mise en oeuvre au moyen d'une fonction énergie définie par :

$$E_{\widehat{S_{E,n,f}}}(t) = \sum_{k=-T_E}^{T_E} \left| \widehat{S_{E,n,f}}(t+k) \right|^2 \qquad (8)$$

Où

$t$ correspond à un instant d'échantillonnage donné,
$2T_E$ est la taille d'une fenêtre temporelle glissante, et
$\widehat{S_{E,n,f}}$ est le signal audio enregistré normalisé filtré obtenu à la fin de l'étape 430).

Au terme de la première sous-étape 510) l'énergie est obtenue sur la fenêtre temporelle de mesure, ceci pour tous les échantillons de sorte qu'une identification « grossière » des instants associés aux évènements vibro-acoustiques $e_{va}$ est déjà possible. Cela étant, ce signal est susceptible de comprendre encore un certain nombre de pics, dits pics secondaires, qui ne correspondent pas auxdits évènement vibro-acoustique $e_{va}$.

A cet égard, l'étape 500) peut comprendre, suite à la première sous-étape 510), une deuxième sous-étape 520) de lissage du signal $E_{\widehat{S_{E,n,f}}}$. Elle est mise en oeuvre au moyen d'une fonction lissage définie par le produit de convolution du signal $E_{\widehat{S_{E,n,f}}}$ avec une fenêtre de pondération de type Hamming :

$$\tilde{E}_{\widehat{S_{E,n,f}}} := E_{\widehat{S_{E,n,f}}} * Hamming\left(T_{lissage}\right) \qquad (9)$$

Où
$\tilde{E}_{\widehat{S_{E,n,f}}}$ est le signal lissé,
$E_{\widehat{S_{E,n,f}}}$ est le signal avant lissage, Hamming est la fenêtre de pondération, et
$T_{lissage}$ est la taille de la fenêtre de pondération.

Cette sous-étape de lissage permet de ne conserver que les pics majeurs dans le signal $E_{\widehat{S_{E,n,f}}}$. En effet, l'utilisation de la fenêtre de pondération de type Hamming permet avantageusement de lisser les valeurs du signal $E_{\widehat{S_{E,n,f}}}$ en appliquant un plus grand coefficient d'observation au centre de la fenêtre et des pondérations de plus en plus faibles à mesure que l'on s'éloigne du centre de la fenêtre. Ainsi, on annule les pics secondaires, tandis que les pics présentant l'amplitude la plus importante sont conservés.

Au terme de la deuxième sous-étape 520) le signal correspondant à l'énergie est lissé.

Avantageusement, l'étape 500) peut comprendre, suite à la deuxième sous-étape 520), une troisième sous-étape 530) d'extraction à partir du signal lissé $\tilde{E}_{\widehat{S_{E,n,f}}}$ de l'ensemble des P instants d'échantillonnage $t_i$ correspondant à des maxima locaux $m_{li}$ appelés encore des candidats de début d'événements vibro-acoustique $e_{va}$.

Lesdits instants d'échantillonnage correspondant au début d'évènements vibro-acoustiques $e_{va}$ extraits sont

tels que :

$$\tilde{E}_{\widehat{S_{E,n,f}}}(t_i) > \tilde{E}_{\widehat{S_{E,n,f}}}(t_{i-1}) \tag{10}$$

$$\tilde{E}_{\widehat{S_{E,n,f}}}(t_i) > \tilde{E}_{\widehat{S_{E,n,f}}}(t_{i+1}) \tag{11}$$

Comme illustré encore sur la figure 6, l'étape 500) comprend, suite à la troisième sous-étape 530), une quatrième sous-étape 540) de présélection des candidats de début d'évènements vibro-acoustique $e_{va}$. Cette quatrième sous-étape 540) vise à éliminer les « paquets » de pics trop rapprochés dans le temps.

Elle est mise en oeuvre au moyen des sous-étapes secondaires 5400) et 5401) identifiées sur la figure 6 par des encadrés en pointillés.

Une première sous-étape secondaire 5400) consiste à effectuer un regroupement de candidats $m_{li}$ associés aux P instants d'échantillonnage $t_j$ selon un premier critère de sélection. Le premier critère de sélection correspond au regroupement des instants d'échantillonnage candidats espacés par un nombre d'échantillons m prédéterminé. Les instants candidats répondant au premier critère de sélections sont alors regroupés en groupes $g_j$,

Une deuxième sous-étape secondaire 5401) consiste à conserver dans chaque groupe $g_j$ les instants associés aux maxima locaux $m_{li}$ pour lesquels la valeur maximale de $\tilde{E}_{\widehat{S_{E,n,f}}}$ est obtenue. De cette manière, pour chaque groupe $g_i$ d'échantillons seul un instant d'échantillonnage $t_i$ est conservé.

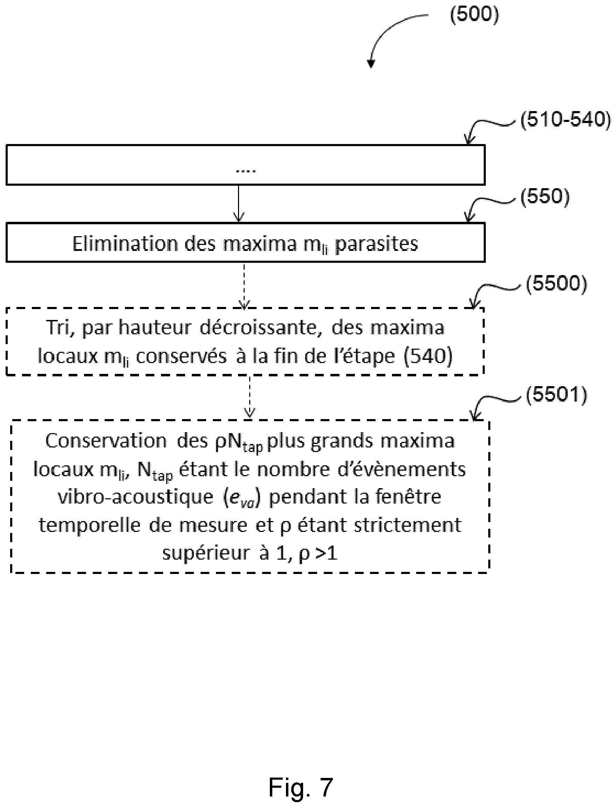

Comme illustré sur la figure 7, l'étape 500) peut comprendre, suite à la quatrième sous-étape 540), une cinquième sous-étape 550) d'élimination des maxima $m_{li}$ parasites. En effet, quand bien même une présélection est effectuée lors de l'étape 540), le signal peut encore comporter des pics parasites qu'il est préférable d'éliminer.

Les pics parasites peuvent être, par exemple, des faux positifs. On entend par « faux positif » des pics qui sont assimilés à des maxima locaux $m_{li}$ alors même qu'ils ne devraient pas l'être. La cinquième sous-étape 550) n'est pas obligatoire, en particulier si le signal ne comporte pas de pics parasites. Cela étant, en pratique, il est fort probable que de tels pics soient présents dans le signal.

La cinquième sous-étape 550) comprend des sous-étapes secondaires 5500) et 5501) identifiées sur la figure par des encadrés en pointillés.

La cinquième sous-étape comprend une première sous-étape 5500) de tri, par hauteur décroissante, des maxima locaux $m_{li}$ conservés à la fin de l'étape 540). Il faut comprend ici, qu'indépendamment des groupes $g_i$ auxquels les maximas $m_{li}$ appartiennent, lesdits maxima $m_{li}$ sont triés par hauteur décroissante.

Ensuite, lors d'une deuxième sous-étape secondaire 5501), des $\rho N_{tap}$ plus grands maxima locaux $m_{li}$, $N_{tap}$ étant le nombre d'évènements vibro-acoustiques $e_{va}$ compris dans la fenêtre temporelle de mesure et $\rho$ étant strictement supérieur à 1 ($\rho$ étant un entier naturel, $\rho > 1$) sont conservés au regard du tri, par hauteur décroissante, effectué lors de l'étape 5500).

La cinquième sous-étape 550) permet une identification plus affinée des instants associés aux évènements vibro-acoustiques $e_{va}$ dans le signal $\tilde{E}_{\widehat{S_{E,n,f}}}$. Les temps de tape de l'utilisateur peuvent être calculés avec une relative précision.

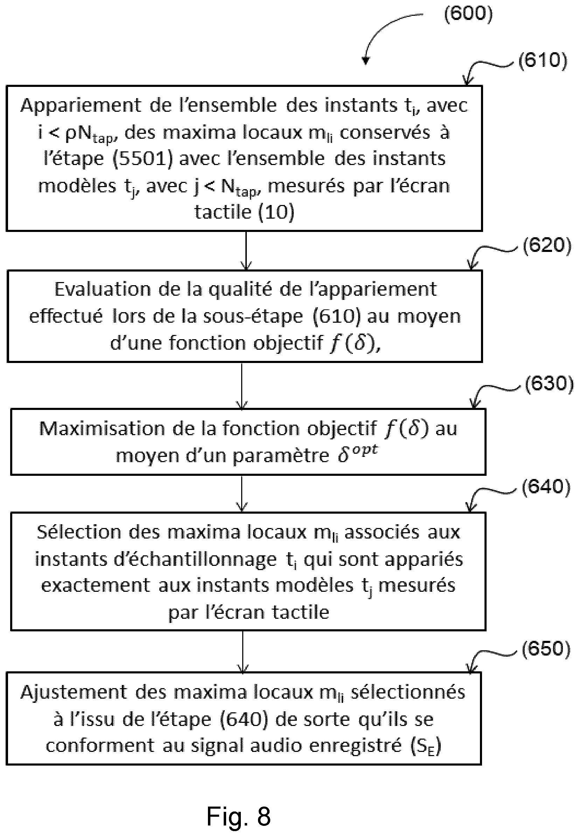

En référence à la figure 8, le procédé selon l'invention comprend en outre une étape d'optimisation du signal résultant de l'étape 500). L'étape d'optimisation vise à identifier précisément les maxima $m_{li}$ correspondant aux évènements vibro-acoustiques $e_{va}$ par un calquage ou une superposition du signal résultant de l'étape 500) avec la mesure des tapes de l'utilisateur effectuée par l'écran tactile 10.

L'étape 600) comprend une première sous-étape 610) d'appariement de l'ensemble des instants $t_i$, avec i < $\rho N_{tap}$, des maxima locaux $m_{li}$ conservés à l'étape 5501) avec l'ensemble des instants modèles $t_j$, avec j < $N_{tap}$, mesurés par l'écran tactile 10. L'appariement permet de mettre en évidence les instants $t_i$ qui correspondent aux instants modèles $t_j$ de la mesure effectuée par l'écran tactile 10 de ceux qui ne correspondent à aucun des instants $t_j$.

Suite à cette première sous-étape 610), il est préférable de procéder à une évaluation 620) de la qualité de l'appariement effectué. La qualité de l'appariement s'apprécie au regard du nombre d'instants $t_i$ qui se « superposent » ou s'apparient avec les instants modèles $t_j$.

La qualité de l'appariement est évaluée au moyen d'une fonction objectif $f(\delta)$ définie par :

$$f(\delta) := \left| \{ j \in [1, N_{tap}] \, t.q. \; matches(\tilde{t_j}, \delta) = 1 \} \right| \tag{12}$$

Où la fonction $matches\left(\widetilde{t_j}, \delta\right)$ est définie par :

$$matches\left(\widetilde{t_j}, \delta\right) := \left|\left\{i \in \left[1, \rho N_{tap}\right] t.q. \quad \widetilde{t} + \rho - t_i < \epsilon\right\}\right| \qquad (13)$$

et ε est une valeur seuil (en millisecondes) qui contrôle la différence maximale tolérée pour considérer que l'appariement est de qualité.

De préférence, ε peut varier de 30 à 100 millisecondes.

L'étape 600) peut en outre comprendre une troisième sous-étape 630) qui consiste à maximiser la fonction objectif $f(\delta)$ au moyen d'un paramètre $\delta^{opt}$.

Le paramètre $\delta^{opt}$ est défini par :

$$\delta^{opt} := argmax_{\delta \in [0, \delta_{max}]} f(\delta) \qquad (14)$$

Où $\delta_{max}$ est une valeur seuil appropriée inférieure à $\widetilde{t} N_{tap}$.

A la fin de la troisième sous-étape 630) il est possible d'évaluer avec une bonne précision la qualité de l'appariement effectué lors de la première sous-étape 610) et de corriger éventuellement cet appariement.

Une quatrième sous-étape de 640) sélection est alors mise en oeuvre. Elle consiste à sélectionner les maxima locaux $m_{li}$ associés aux instants d'échantillonnage $t_i$ qui sont appariés exactement aux instants modèles $t_j$ mesuré par l'écran tactile.

A la fin de cette étape, le signal $\widetilde{E}_{\widehat{S_{E,n,f}}}$ est en principe épuré de tous les pics indésirables et les pics correspondant aux évènements vibro-acoustiques $e_{va}$ sont appariés avec les mesures de l'écran tactile 10. En d'autres termes, les instants $t_i$ des maxima $m_{li}$ correspondent aux instant $t_j$ des mesures de l'écran tactile 10.

L'étape 600) peut comprendre, suite à la quatrième sous-étape 640), une cinquième sous-étape 650) d'ajustement dans laquelle les maxima locaux $m_{li}$ sélectionnés à l'issu de l'étape 640) sont ajustés de sorte à se conformer au signal d'origine, c'est-à-dire au signal audio enregistré $S_E$. En effet, les étapes de lissage et de filtrage réalisées au cours des étapes 300) et 400) ont pu introduire un déphasage du signal qu'il est nécessaire de compenser.

Cette quatrième étape 640) d'ajustement est donc effectuée en appliquant une fonction de compensation de déphasage aux maxima $m_{li} = \widetilde{E}_{\widehat{S_{E,n,f}}}(t_i)$. Ladite fonction de compensation de déphasage étant définie par :

$$\delta^{opt} := argmax_{\delta \in [0, \delta_{max}]} f(\delta) \qquad (15)$$

Où $t$

$_i$ sont les instants d'échantillonnage associé aux maxima locaux $m_{li}$ sélectionnés à l'issu de l'étape 640),
$T_i$ étant le nombre d'échantillons compris dans la fenêtre temporelle de recherche, et
$E_{\widehat{S_{E,n,f}}}$ est le signal avant lissage.

A l'issu de l'étape 600) d'optimisation, les temps de tapes de l'utilisateur en réponse au stimulus auditif, i.e. signal audio de référence $S_R$, sont déterminés avec une très bonne précision.

Dans un autre mode de réalisation de la présente invention, il est possible de déterminer les temps de tape d'un utilisateur sans pour autant que celui-ci effectue des tapes sur l'écran tactile 10 en réponse à un stimulus auditif. Dans ce mode de réalisation, il ne serait alors pas nécessaire d'effectuer les étapes de synchronisation du signal audio de référence $S_R$ avec le signal audio enregistré ni même d'effectuer l'identification, i.e. la localisation du début, dudit signal de référence $S_R$ dans le signal enregistré $S_E$.

## Revendications

1. Procédé de détermination des temps de tape d'un utilisateur en réponse à un stimulus auditif, le procédé comprenant les étapes suivantes :

- 100) lecture du stimulus auditif, ledit stimulus auditif correspondant au signal audio de référence ($S_R$),
- 200) enregistrement du signal audio de référence ($S_R$) et d'un ou plusieurs évènements vibro-acoustique ($e_{va}$), lesdits évènements vibro-acoustiques ($e_{va}$) faisant suite à une ou plusieurs tapes de l'utilisateur sur un écran tactile (10) d'un dispositif (1) à écran tactile en réaction au signal audio de référence ($S_R$), le signal résultant de l'enregistrement étant appelé signal audio enregistré ($S_E$),
- 300) détection du signal audio de référence ($S_R$) dans le signal audio enregistré ($S_E$),
- le signal audio enregistré ($S_E$) est enregistré au moyen d'un microphone (30) du dispositif (1) à écran tactile,

le procédé étant **caractérisé en ce que**:

- lors de l'étape de détection 300) du signal audio de référence ($S_R$) dans le signal audio enregistré ($S_E$), le signal audio de référence ($S_R$) et le signal audio enregistré ($S_E$) sont placés sur une échelle temporelle commune unique,
- il comprend une étape 400) de filtrage dans laquelle le signal enregistré obtenu subséquemment à l'étape 300) de détection, est préalablement filtré, puis normalisé de manière à conserver uniquement les fréquences correspondant aux évènements vibro-acoustiques ($e_{va}$) générés par les actions de l'utilisateur sur l'écran tactile, et **en ce que**
- il comprend une étape 500) de détection des évènements vibro-acoustiques ($e_{va}$) dans laquelle des instants ($t_i$) associés aux évènements vibro-acoustiques ($e_{va}$) sont identifiés dans le signal obtenu à la fin de l'étape 400) de filtrage.

2.  Procédé selon la revendication 1, dans lequel l'étape 300) comprend une première sous-étape 310) de normalisation du signal audio enregistré ($S_E$) de sorte à obtenir un signal audio enregistré normalisé ($S_{E,n}$).

3.  Procédé selon la revendication 2, dans lequel l'étape 300) comprend une deuxième sous-étape 320) dans laquelle le signal audio de référence ($S_R$) est ré-échantillonné au même taux que le signal audio enregistré ($S_E$) et normalisé de sorte à obtenir un signal audio de référence ré-échantillonné et normalisé ($S_{R,n}$).

4.  Procédé selon l'une des revendications 2 à 3, dans lequel l'opération de normalisation réalisée lors de l'étape 300) est effectuée au moyen d'une fonction de normalisation définie par :

$$f_n(X) := \frac{X - moyenne\,(X)}{médiane\,\big(abs(X)\big)}$$

Où X est le signal audio enregistré ($S_E$) ou le signal audio de référence ($S_R$) ré-échantillonné, moyenne (X) est la valeur moyenne du signal considéré, abs (X) est le signal dont les échantillons sont des valeurs absolues des échantillons du signal X et médiane (abs(X)) est la valeur médiane du signal abs (X).

5.  Procédé selon l'une des revendications 3 à 4, dans lequel l'étape 300) comprend, suite à la première et la deuxième sous-étape 320), une troisième sous-étape 330) d'identification du début du signal audio de référence ré-échantillonné et normalisé ($S_{R,n}$) dans le signal audio enregistré normalisé ($S_{E,n}$).

6.  Procédé selon la revendication 5, dans lequel la troisième sous-étape 330) comprend les sous-étapes suivantes :

- 332) construction d'un filtre adapté de sorte à identifier dans le signal audio enregistré normalisé ($S_{E,n}$) l'instant d'échantillonnage ($t_{SR}$) le plus probable qui correspond au début des K premiers échantillons du signal audio de référence ré-échantillonné et normalisé ($S_{R,n}$),
- 334) détermination d'une fenêtre temporelle de taille ($T_{SR,n}$) dans laquelle effectuer la recherche du signal audio de référence ré-échantillonné normalisé ($S_{R,n}$) dans le signal audio enregistré normalisé ($S_{E,n}$),
- 336) détermination de l'instant d'échantillonnage ($t_{SR}$).

7.  Procédé selon la revendication 6, dans lequel l'instant d'échantillonnage ($t_{SR}$) est défini de la manière qui suit :

$$t_{SR} := argmax_{t<T_{SR,n}} \sum_{k=t}^{t+K} S_{E,n}(k) \cdot S_{R,n}(k-t)$$

Où (t) correspond à un instant d'échantillonnage donné, ($t_{SR}$) exprime l'instant d'échantillonnage correspondant au début signal audio de référence ré-échantillonné et normalisé ($S_{R,n}$) dans le signal audio enregistré normalisé ($S_{E,n}$), ($T_{SR,n}$) est la taille de la fenêtre temporelle (en secondes) et argmax, désigne l'ensemble des points, sur la fenêtre temporelle ($T_{SR,n}$), en lesquels le produit de convolution $\sum_{k=t}^{t+K} S_{E,n}(k) \cdot S_{R,n}(k-t)$ atteint sa valeur maximale.

8. Procédé selon l'une quelconque des revendications précédentes, dans lequel l'étape 400) de filtrage comprend une première sous-étape de filtrage 410) et optionnellement une deuxième sous-étape de filtrage 420).

9. Procédé selon la revendication 8, dans lequel :

    - la première sous-étape de filtrage 410) est mise en oeuvre au moyen d'un filtre passe-bande de type Butterworth d'ordre 1 ayant une fréquence basse de 50 Hz et une fréquence haute de 200 Hz, et
    - la seconde sous-étape de filtrage 420) est mise en oeuvre au moyen d'un filtre passe-bas de type Butterworth d'ordre 1 ayant une fréquence haute de 400 Hz,

de sorte qu'à la fin de la deuxième sous-étape 420) on obtient un signal audio enregistré normalisé filtré ($S_{E,n,f}$).

10. Procédé selon l'une des revendications 8 à 9, dans lequel suite à la deuxième sous-étape 420), l'étape 400) comprend une troisième sous-étape 430) de normalisation locale du signal audio enregistré normalisé filtré ($S_{E,n,f}$) obtenu à la fin de l'étape 420) de sorte à obtenir un signal audio enregistré normalisé filtré ($\widehat{S_{E,n,f}}$), ladite sous-étape de normalisation locale 430) étant effectuée au moyen d'une fonction de normalisation locale définie par :

$$f_{nloc}(S_{E,n,f}) := \frac{S_{E,n,f} - moyenne_{loc}(S_{E,n,f})}{moyenne_{loc}(abs_{loc}(S_{E,n,f} - moyenne_{loc}(S_{E,n,f})))}$$

Où ($S_{E,n,f}$) est le signal audio enregistré normalisé filtré, $moyenne_{loc}(S_{E,n,f})$ est la valeur moyenne locale du signal audio enregistré normalisé filtré ($S_{E,n,f}$), $abs_{loc}(S_{E,n,f} - moyenne_{loc}(S_{E,n,f}))$ est le signal dont les échantillons sont des valeurs absolues locales des échantillons du signal audio enregistré normalisé filtré ($S_{E,n,f}$) sur le sous-ensemble d'une fenêtre glissante de taille $2T_{norm}$.

11. Procédé selon l'une quelconque des revendications 8 à 10, dans lequel suite à l'étape 400) de filtrage on met en oeuvre l'étape 500) de détection des évènements vibro-acoustiques ($e_{va}$).

12. Procédé selon la revendication 11, dans lequel l'étape 500) comprend une première sous-étape 510) de détermination de l'énergie ($E_{\widehat{S_{E,n,f}}}$) du signal audio enregistré normalisé filtré ($S_{E,n,f}$), ladite première sous-étape 510) étant effectuée au moyen d'une fonction énergie définie par :

$$E_{\widehat{S_{E,n,f}}}(t) = \sum_{k=-T_E}^{T_E} \left| \widehat{S_{E,n,f}}(t+k) \right|^2$$

Où (t) correspond à un instant d'échantillonnage donné, ($2T_E$) est la taille d'une fenêtre temporelle glissante, le nombre d'échantillons étant égal $2T_E$ dans ladite fenêtre glissante, et ($\widehat{S_{E,n,f}}$) est le signal audio enregistré normalisé filtré obtenu à la fin de l'étape 430).

**13.** Procédé selon la revendication 12, dans lequel l'étape 500) comprend, suite à la première sous-étape 510), une deuxième sous-étape 520) de lissage du signal ( $E_{\widehat{S_{E,n,f}}}$ ) obtenu à la fin de la première sous-étape 510) au moyen d'une fonction lissage définie par le produit de convolution du signal ( $E_{\widehat{S_{E,n,f}}}$ ) avec une fenêtre de pondération de type Hamming :

$$\tilde{E}_{\widehat{S_{E,n,f}}} := E_{\widehat{S_{E,n,f}}} * Hamming\ (T_{lissage})$$

Où ( $\tilde{E}_{\widehat{S_{E,n,f}}}$ ) est le signal lissé, ( $E_{\widehat{S_{E,n,f}}}$ ) est le signal avant lissage, Hamming est la fenêtre de pondération et ($T_{lissage}$) est la taille de la fenêtre de pondération.

**14.** Procédé selon la revendication 13, dans lequel l'étape 500) comprend, suite à la deuxième sous-étape 520, une troisième sous-étape 530) d'extraction à partir du signal lissé ( $\tilde{E}_{\widehat{S_{E,n,f}}}$ ) de l'ensemble des P instants d'échantillonnage ($t_i$) correspondant à des maxima locaux ($m_{li}$) et/ou candidats de début d'évènements vibro-acoustiques ($e_{va}$), lesdits instants d'échantillonnage étant tels que

$$\tilde{E}_{\widehat{S_{E,n,f}}}\ (t_i) > \tilde{E}_{\widehat{S_{E,n,f}}}\ (t_{i-1})$$

et

$$\tilde{E}_{\widehat{S_{E,n,f}}}\ (t_i) > \tilde{E}_{\widehat{S_{E,n,f}}}\ (t_{i+1})$$

**15.** Procédé selon la revendication 14, dans lequel l'étape 500) comprend, suite à la troisième sous-étape 530), une quatrième sous-étape 540) de présélection des candidats de début d'évènements vibro-acoustique ($e_{va}$) comprenant les sous-étapes suivantes :

- 5400) regroupement de candidats ($m_{li}$) associés aux P instants d'échantillonnage ($t_i$) selon un premier critère de sélection, ledit premier critère de sélection correspondant au regroupement des instants d'échantillonnage candidats espacés par un nombre d'échantillons m prédéterminée, de sorte à former des groupes ($g_j$) de maxima locaux ($m_{li}$),
- 5401) conservation dans chaque groupe $g_j$ les instants associés aux maxima locaux ($m_{li}$) pour lesquels la valeur maximale de $\tilde{E}_{\widehat{S_{E,n,f}}}$ est obtenue.

**16.** Procédé selon la revendication 15, dans lequel l'étape 500) comprend, suite à la quatrième sous-étape 540), une cinquième sous-étape 550) d'élimination des maxima ($m_{li}$) parasites comprenant les sous-étapes suivantes :

- 5500) tri, par hauteur décroissante, des maxima locaux ($m_{li}$) conservés à la fin de la quatrième sous-étape 540),
- 5501) conservation des $\rho N_{tap}$ plus grands maxima locaux ($m_{li}$), $N_{tap}$ étant le nombre d'évènements vibro-acoustiques ($e_{va}$) compris dans la fenêtre temporelle de mesure et $\rho$ étant strictement supérieur à 1, $\rho > 1$.

**17.** Procédé selon la revendication 16, comprenant en outre, suite à l'étape 500) de détection des évènements vibro-acoustique ($e_{va}$), une étape supplémentaire 600) d'optimisation du signal obtenu à la fin de l'étape 500), ladite étape comprenant les sous-étapes suivantes :

- 610) appariement de l'ensemble des instants ($t_i$), avec $i < \rho N_{tap}$, des maxima locaux ($m_{li}$) conservés à l'étape 5502) avec l'ensemble des instants modèles ($t_j$), avec $j < N_{tap}$, mesurés par l'écran tactile (10),
- 620) évaluation de la qualité de l'appariement effectué lors de la sous-étape 610) au moyen d'une fonction objectif ($f(\delta)$),
- 630) maximisation de la fonction objectif ($f(\delta)$) au moyen d'un paramètre ($\delta^{opt}$),

- 640) sélection des maxima locaux ($m_{li}$) associés aux instants d'échantillonnage ($t_i$) qui sont appariés exactement aux instants modèles ($t_j$) mesurés par l'écran tactile.

**18.** Procédé selon la revendication 17, dans lequel la fonction objectif est définie par :

$$f(\delta) := \left|\left\{ j \, \epsilon \, \left[1, N_{tap}\right] t.\,q. \quad matches\left(\widetilde{t_j}, \delta\right) = 1 \right\}\right|$$

Où la fonction $matches\left(\widetilde{t_j}, \delta\right)$ est définie par :

$$matches\left(\widetilde{t_j}, \delta\right) := \left|\left\{ i \, \in \, \left[1, \rho N_{tap}\right] t.\,q. \quad \tilde{t} + \rho - t_i < \epsilon \right\}\right|$$

et ($\varepsilon$) est une valeur seuil (en millisecondes) qui contrôle la différence maximale tolérée pour considérer que l'appariement est de qualité.

**19.** Procédé selon l'une des revendications 17 à 18, dans lequel le paramètre ($\delta^{opt}$) est défini par :

$$\delta^{opt} := argmax_{\delta \in [0, \delta_{max}]} f(\delta)$$

Où $\delta_{max}$ est une valeur seuil appropriée inférieure à $\tilde{t}N_{tap}$.

**20.** Procédé selon l'une quelconque des revendications 17 à 19, dans lequel l'étape 600) comprend, suite à la quatrième sous-étape 640), une cinquième sous-étape 650) d'ajustement dans laquelle les maxima locaux ($m_{li}$) sélectionnés à l'issu de l'étape 640) sont ajustés de sorte à se conformer au signal audio enregistré ($S_E$).

**21.** Procédé selon la revendication 20, dans lequel l'ajustement est effectué en appliquant une fonction de compensation de déphasage aux maxima $m_{li} = \tilde{E}_{\widehat{S_{E,n,f}}}(t_i)$, ladite fonction étant définie par :

$$\widehat{t_{m_{li}}} := argmax_{t \in [t_i - T_i, \, t_i + T_i]} E_{\widehat{S_{E,n,f}}}(t_i)$$

Où ($t_i$) est l'instant d'échantillonnage associé aux maxima locaux $m_{li}$ sélectionnés à l'issu de l'étape 560), ($T_i$) étant le nombre d'échantillons compris dans la fenêtre temporelle de recherche, ($E_{\widehat{S_{E,n,f}}}$) est le signal avant lissage.

**22.** Dispositif (1) à écran tactile adapté pour la mise en oeuvre du procédé selon l'une quelconque des revendications 1 à 21, ledit dispositif comprenant :

- un écran tactile (10),
- un microphone (30),
- une unité centrale de traitement configurée pour exécuter au moins les étapes du procédé selon l'une des revendications 1 à 21.

**23.** Programme d'ordinateur comprenant des instructions qui lorsque le programme est exécuté par l'ordinateur conduisent celui-ci à mettre en oeuvre le procédé selon l'une quelconque des revendications 1 à 21.

**24.** Moyen de stockage pour ordinateur sur lequel est enregistré le programme d'ordinateur selon la revendication 23.

**Patentansprüche**

**1.** Verfahren zur Bestimmung der Tippzeiten eines Benutzers als Reaktion auf einen auditiven Stimulus, wobei das Verfahren die folgenden Schritte umfasst:

- 100) Lesung des auditiven Stimulus, wobei der auditive Stimulus dem Referenzaudiosignal ($S_R$) entspricht,
- 200) Aufzeichnung des Referenzaudiosignals (SR) und eines oder mehrerer vibroakustischer Ereignisse ($e_{va}$), wobei die vibroakustischen Ereignisse ($e_{va}$) einem oder mehreren Tippen des Benutzers als Reaktion auf ein Referenzaudiosignal (SR) auf einem Berührungsbildschirm (10) einer Vorrichtung (1) mit Berührungsbildschirm folgen, wobei das aus der Aufzeichnung resultierende Signal als aufgezeichnetes Audiosignal($S_E$) bezeichnet wird,
- 300) Erfassung des Referenzaudiosignals($S_R$) im aufgezeichneten Audiosignal (SE),
- wobei das aufgezeichnete Audiosignal ($S_E$) mittels eines Mikrofons (30) der Vorrichtung (1) mit Berührungsbildschirm aufgezeichnet wird;

wobei das Verfahren **dadurch gekennzeichnet ist, dass**:

- das Referenzaudiosignal ($S_R$) und das aufgezeichnete Audiosignal ($S_E$) beim Schritt der Erfassung 300) des Referenzaudiosignals ($S_R$) im aufgezeichneten Audiosignal ($S_E$) auf eine einzige gemeinsame zeitliche Skala platziert werden,
- es einen Schritt 400) der Filterung umfasst, bei dem das im Anschluss an den Schritt 300) der Erfassung erhaltene aufgezeichnete Signal vorab gefiltert, dann derart normalisiert wird, dass nur die Frequenzen beibehalten werden, die den vibroakustischen Ereignissen ($e_{va}$) entsprechen, die durch die Aktionen des Benutzers auf dem Berührungsbildschirm erzeugt werden, und dadurch, dass

es einen Schritt 500) der Erfassung der vibroakustischen Ereignisse($e_{va}$) umfasst, bei dem die den vibroakustischen Ereignissen ($e_{va}$) zugeordneten Instanzen ($t_i$) im Signal identifiziert werden, das am Ende des Schritts 400) der Filterung erhalten wird.

2. Verfahren nach Anspruch 1, wobei der Schritt 300) einen ersten Teilschritt 310) der Normalisierung des aufgezeichneten Audiosignals (SE) umfasst, sodass ein normalisiertes aufgezeichnetes Audiosignal ($S_{E,n}$) erhalten wird.

3. Verfahren nach Anspruch 2, wobei der Schritt 300) einen zweiten Teilschritt 320) umfasst, bei dem das Referenzaudiosignal (SR) mit der gleichen Rate wie das aufgezeichnete Audiosignal (SE) erneut abgetastet wird und normalisiert wird, sodass ein erneut abgetastetes und normalisiertes Referenzaudiosignal ($S_{R,n}$) erhalten wird.

4. Verfahren nach einem der Ansprüche 2 bis 3, wobei der beim Schritt 300) ausgeführte Vorgang der Normalisierung mithilfe einer Normalisierungsfunktion durchgeführt wird, die definiert ist durch:

$$f_n(X) := \frac{X - Durchschnitt\ (X)}{median\left(abs(X)\right)}$$

wobei X das aufgezeichnete Audiosignal ($S_E$) oder das erneut abgetastete Referenzaudiosignal ($S_R$) ist, Durchschnitt (X) der Durchschnittswert des betrachteten Signals ist, abs (X) das Signal ist, dessen Abtastungen absolute Werte der Abtastungen der Signals X sind und Median (abs(X)) der Medianwert des Signals abs (X) ist.

5. Verfahren nach einem der Ansprüche 3 bis 4, wobei der Schritt 300) im Anschluss an den ersten und den zweiten Teilschritt 320) einen dritten Teilschritt 330) der Identifizierung des Anfangs des erneut abgetasteten und normalisierten Referenzaudiosignals ($S_{R,n}$) im normalisierten aufgezeichneten Audiosignal ($S_{E,H}$) umfasst.

6. Verfahren nach Anspruch 5, wobei der dritte Teilschritt 330) die folgenden Teilschritte umfasst:

- 332) Aufbau eines Filters, der dazu angepasst ist, im normalisierten aufgezeichneten Audiosignal($S_{E,n}$) den wahrscheinlichsten Abtastzeitpunkt ($t_{SR}$) zu identifizieren, der dem Anfang der ersten K Abtastungen des erneut abgetasteten und normalisierten Referenzaudiosignals ($S_{R,n}$) entspricht,
- 334) Bestimmung eines Zeitfensters der Größe ($T_{SR,n}$), in dem die Suche nach dem erneut abgetasteten normalisierten Referenzaudiosignal ($S_{R,n}$) im normalisierten aufgezeichneten Audiosignal ($S_{E,n}$) durchzuführen ist,
- 336) Bestimmung des Abtastzeitpunkts ($t_{SR}$).

7. Verfahren nach Anspruch 6, wobei der Abtastzeitpunkt ($t_{SR}$) auf folgende Weise definiert ist:

$$t_{SR} := argmax_{t < T_{SR,n}} \sum_{k=t}^{t+K} S_{E,n}(k) \cdot S_{R,n}(k-t)$$

wobei (t) einem gegebenen Abtastzeitpunkt entspricht, (t_{SR}) den Abtastzeitpunkt ausdrückt, der dem Anfang des erneut abgetasteten und normalisierten Referenzaudiosignals (S_{R, n}) im normalisierten aufgezeichneten Audiosignal (S_{E,n}) entspricht, (t_{SR,n}) die Größe des Zeitfensters (in Sekunden) ist, und argmax die Gesamtheit der Punkte auf dem Zeitfenster (t_{SR,n}) bezeichnet, bei denen das Konvolutionsprodukt $\sum_{k=t}^{t+K} S_{E,n}(k) \cdot S_{R,n}(k-t)$ seinen Höchstwert erreicht.

8. Verfahren nach einem der vorstehenden Ansprüche, wobei der Schritt 400) der Filterung einen ersten Teilschritt der Filterung 410) und wahlweise einen zweiten Teilschritt der Filterung 420) umfasst.

9. Verfahren nach Anspruch 8, wobei:

- der erste Teilschritt der Filterung 410) mittels eines Bandpassfilters vom Butterworth-Typ der Ordnung 1, der eine tiefe Frequenz von 50 Hz und eine hohe Frequenz von 200 Hz aufweist, eingesetzt wird, und
- der zweite Teilschritt der Filterung 420) mittels eines Tiefpassfilters vom Butterworth-Typ der Ordnung 1, der eine hohe Frequenz von 400 Hz aufweist, eingesetzt wird,

sodass am Ende des zweiten Teilschritts 420) ein gefiltertes normalisiertes aufgezeichnetes Audiosignal (S_{E,n,f}) erhalten wird.

10. Verfahren nach einem der Ansprüche 8 bis 9, wobei im Anschluss an den zweiten Teilschritt 420) der Schritt 400) einen dritten Teilschritt 430) der lokalen Normalisierung des gefilterten normalisierten aufgezeichneten Audiosignals (S_{E,n,f}), das am Ende des Schritts 420) erhalten wird, umfasst, sodass ein gefiltertes normalisiertes aufgezeichnetes Audiosignal $\widetilde{S_{E,n,f}}$ ), erhalten wird, wobei der Teilschritt der lokalen Normalisierung 430) mittels einer lokalen Normalisierungsfunktion durchgeführt wird, die definiert ist durch:

$$f_{nloc}(S_{E,n,f}) := \frac{S_{E,n,f} - Durchschnitt_{loc}(S_{E,n,f})}{Durchschnitt_{loc}(abs_{loc}(S_{E,n,f} - Durchschnitt_{loc}(S_{E,n,f})))}$$

wobei (S_{E,n,f}) das gefilterte normalisierte aufgezeichnete Audiosignal ist, Durchschnitt_{loc}(S_{E,n,f}) der lokale Durchschnittswert des gefilterten normalisierten aufgezeichneten Audiosignals (S_{E,n,f}) ist, abs_{loc}(S_{E,n,f} - Durchschnitt_{loc}(S_{E,n,f})) das Signal ist, dessen Abtastungen lokale absolute Werte der Abtastungen des gefilterten normalisierten aufgezeichneten Audiosignals (S_{E,n,f}) auf der Teilgesamtheit eines gleitenden Fensters der Größe 2T_{norm} sind.

11. Verfahren nach einem der Ansprüche 8 bis 10, wobei im Anschluss an den Schritt 400) der Filterung der Schritt 500) der Erfassung der vibroakustischen Ereignisse (e_{va}) eingesetzt wird.

12. Verfahren nach Anspruch 11, wobei der Schritt 500) einen ersten Teilschritt 510) der Bestimmung der Energie ($E_{\widetilde{S_{E,n,f}}}$) des gefilterten normalisierten aufgezeichneten Audiosignals ($\widetilde{S_{E,n,f}}$) umfasst, wobei der erste Teilschritt 510) mittels einer Energiefunktion durchgeführt wird, die definiert ist durch:

$$E_{\widetilde{S_{E,n,f}}}(t) = \sum_{k=-T_E}^{T_E} \left|\widetilde{S_{E,n,f}}(t+k)\right|^2$$

wobei (t) einem gegebenen Abtastzeitpunkt entspricht, (2T_E) die Größe eines gleitenden Zeitfensters ist, wobei die Anzahl von Abtastungen im gleitenden Fenster gleich 2T_E ist und das am Ende des Schritts 430) erhaltene gefilterte normalisierte aufgezeichnete Audiosignal ist.

13. Verfahren nach Anspruch 12, wobei der Schritt 500) im Anschluss an den ersten Teilschritt 510) einen zweiten Teilschritt 520) der Glättung des am Ende des ersten Teilschritts 510) erhaltenen Signals ( $E_{\widehat{S_{E,n,f}}}$ ) mittels einer Glättungsfunktion umfasst, die durch das Konvolutionprodukt des Signals ( $E_{\widehat{S_{E,n,f}}}$' ) mit einem Gewichtungsfenster vom Hamming-Typ definiert ist:

$$\tilde{E}_{\widehat{S_{E,n,f}}} := E_{\widehat{S_{E,n,f}}} * Hamming \; (T_{Glättung})$$

wobei ( $\tilde{E}_{\widehat{S_{E,n,f}}}$ ) das geglättete Signal ist, ( $E_{\widehat{S_{E,n,f}}}$ ) das Signal vor der Glättung ist, Hamming das Gewichtungsfenster ist, und ($T_{Glättung}$) die Größe des Gewichtungsfensters ist.

14. Verfahren nach Anspruch 13, wobei der Schritt 500) im Anschluss an den zweiten Teilschritt 520 einen dritten Teilschritt 530) der Extraktion ausgehend von dem geglätteten Signal ( $\tilde{E}_{\widehat{S_{E,n,f}}}$ ) der Gesamtheit der P Abtastzeitpunkte ($t_i$) umfasst, die lokalen Maxima ($m_{li}$) und/oder Kandidaten des Anfangs vibroakustischer Ereignisse ($e_{va}$) entsprechen, wobei die Abtastzeitpunkte derart sind, dass

$$\tilde{E}_{\widehat{S_{E,n,f}}} \; (t_i) > \tilde{E}_{\widehat{S_{E,n,f}}} \; (t_{i-1})$$

und

$$\tilde{E}_{\widehat{S_{E,n,f}}} \; (t_i) > \tilde{E}_{\widehat{S_{E,n,f}}} \; (t_{i+1})$$

15. Verfahren nach Anspruch 14, wobei der Schritt 500) im Anschluss an den dritten Teilschritt 530) einen vierten Teilschritt 540) der Vorauswahl der Kandidaten des Anfangs vibroakustischer Ereignisse ($e_{va}$) umfasst, der die folgenden Teilschritte umfasst:

- 5400) Umgruppierung von Kandidaten ($m_{li}$), die den P Abtastzeitpunkten ($t_i$) zugeordnet sind, gemäß einem ersten Auswahlkriterium, wobei das erste Auswahlkriterium der Umgruppierung der Kandidaten-Abtastzeitpunkte entspricht, die durch eine vorbestimmte Anzahl m von Abtastungen beabstandet sind, sodass Gruppen ($g_j$) lokaler Maxima ($m_{li}$) gebildet werden,
- 5401) Beibehaltung, in jeder Gruppe $g_j$, der Zeitpunkte, die den lokalen Maxima ($m_{li}$) zugeordnet sind, für die der Höchstwert von $\tilde{E}_{\widehat{S_{E,n,f}}}$ erhalten wird.

16. Verfahren nach Anspruch 15, wobei der Schritt 500) im Anschluss an den vierten Teilschritt 540) einen fünften Teilschritt 550) der Eliminierung der parasitären Maxima ($m_{li}$) umfasst, der die folgenden Teilschritte umfasst:

- 5500) Ordnung, in absteigender Reihenfolge, der lokalen Maxima ($m_{li}$), die am Ende des vierten Teilschritts 540) beibehalten werden,
- 5501) Beibehaltung der $\rho N_{tap}$ größten lokalen Maxima ($m_{li}$), wobei $N_{tap}$ die Anzahl von vibroakustischen Ereignissen ($e_{va}$) ist, die im Messzeitfenster umfasst sind, und wobei $\rho$ streng größer als 1 ist, $\rho > 1$.

17. Verfahren nach Anspruch 16, weiter umfassend, im Anschluss an den Schritt 500) der Erfassung der vibroakusti-schen Ereignisse ($e_{va}$), einen zusätzlichen Schritt 600) der Optimierung des am Ende des Schritts 500) erhaltenen Signals, wobei der Schritt die folgenden Teilschritte umfasst:

- 610) Paarung der Gesamtheit der Zeitpunkte ($t_i$), mit $i < \rho N_{tap}$, der lokalen Maxima ($m_{li}$), die im Schritt 5502) beibehalten werden, mit der Gesamtheit der Modellzeitpunkte ($t_j$), mit $j < N_{tap}$, die von dem Berührungsbildschirm (10) gemessen werden,
- 620) Auswertung der beim Schritt 610) durchgeführten Paarung mittels einer Zielfunktion (f($\delta$)),
- 630) Maximierung der Zielfunktion (f($\delta$)) mittels eines Parameters ($\delta^{opt}$),
- 640) Auswahl der lokalen Maxima ($m_{li}$), die an Abtastzeitpunkten ($t_i$) zugeordnet sind, die genau mit den von dem Berührungsbildschirm gemessenen Modellzeitpunkten ($t_j$) gepaart sind.

18. Verfahren nach Anspruch 17, wobei die Zielfunktion definiert ist durch:

$$f(\delta) := \left| \left\{ j \in [1, N_{tap}] t.q. \quad matches(\widetilde{t_j}, \delta) = 1 \right\} \right|$$

wobei die Funktion $matches(\widetilde{t_j}, \delta)$ definiert ist durch:

$$matches(\widetilde{t_j}, \delta) := \left| \left\{ i \in [1, \rho N_{tap}] t.q. \quad \widetilde{t} + \rho - t_i < \epsilon \right\} \right|$$

und ($\epsilon$) ein Schwellenwert (in Millisekunden) ist, der die maximale Differenz steuert, die toleriert wird, um die Paarung als qualitätsvoll zu betrachten.

19. Verfahren nach einem der Ansprüche 17 bis 18, wobei der Parameter ($\delta^{opt}$) definiert ist durch:

$$\delta^{opt} := argmax_{\delta \in [0, \delta_{max}]} f(\delta)$$

wobei $\delta_{max}$ ein geeigneter unterer Schwellenwert zu $\widetilde{t} N_{tap}$ ist.

20. Verfahren nach einem der Ansprüche 17 bis 19, wobei der Schritt 600) im Anschluss an den vierten Teilschritt 640) einen fünften Teilschritt 650) der Einstellung umfasst, bei dem die lokalen Maxima ($m_{li}$), die beim Abschluss des Schrittes 640) ausgewählt werden, so eingestellt werden, dass sie mit dem ausgezeichneten Audiosignal ($S_E$) konform sind.

21. Verfahren nach Anspruch 20, wobei die Einstellung durchgeführt wird, indem eine Funktion zur Kompensierung der Phasenverschiebung an den Maxima $m_{li} = \widetilde{E}_{\widehat{S_{E,n,f}}}(t_i)$ angewendet wird, wobei die Funktion definiert ist durch:

$$\widehat{t_{m_{li}}} := argmax_{t \in [t_i - T_i, \ t_i + T_i]} E_{\widehat{S_{E,n,f}}}(t_i)$$

wobei ($t_i$) der Abtastzeitpunkt ist, der den lokalen Maxima $m_{li}$ zugeordnet ist, die beim Abschluss des Schrittes 560) ausgewählt werden, wobei ($t_i$) die Anzahl der Abtastungen ist, die im Suchzeitfenster umfasst sind, $\left( E_{\widehat{S_{E,n,f}}} \right)$ das Signal vor der Glättung ist.

22. Vorrichtung (1) mit Berührungsbildschirm, die für den Einsatz des Verfahrens nach einem der Ansprüche 1 bis 21 angepasst ist, wobei die Vorrichtung umfasst:

   - einen Berührungsbildschirm (10),
   - ein Mikrofon (30),
   - eine zentrale Verarbeitungseinheit, die konfiguriert ist, um mindestens die Schritte des Verfahrens nach einem der Ansprüche 1 bis 21 auszuführen.

23. Computerprogramm, umfassend Anweisungen, die, wenn das Programm von einem Computer ausgeführt wird, diesen veranlassen, das Verfahren nach einem der Ansprüche 1 bis 21 einzusetzen.

24. Speichermittel für einen Computer, auf dem das Computerprogramm nach Anspruch 23 aufgezeichnet ist.

**Claims**

1. A method for determining the tap times of a user in response to an auditory stimulus, the method comprising the following steps:

   - 100) playing back the auditory stimulus, said auditory stimulus corresponding to the reference audio signal $(S_R)$,
   - 200) recording the reference audio signal $(S_R)$ and one or more vibro-acoustic events $(e_{va})$, said vibro-acoustic events $(e_{va})$ being subsequent to one or more taps by the user on a touchscreen (10) of a touchscreen device (1) in reaction to the reference audio signal $(S_R)$, the signal resulting from the recording being referred to as recorded audio signal $(S_E)$,
   - 300) detecting the reference audio signal $(S_R)$ in the recorded audio signal $(S_E)$,
   - the recorded audio signal $(S_E)$ is recorded by means of a microphone (30) of the touchscreen device (1),

   the method being **characterized in that**:

   - during the step of detecting 300) the reference audio signal $(S_R)$ in the recorded audio signal $(S_E)$, the reference audio signal $(S_R)$ and the recorded audio signal $(S_E)$ are placed on a single common time scale,
   - it comprises a filtering step 400) in which the recorded signal obtained subsequently to the detecting step 300) is initially filtered, then normalized in such a way as to keep only the frequencies corresponding to the vibro-acoustic events $(e_{va})$ generated by the actions of the user on the touchscreen, and **in that**
   - it comprises a step 500) of detecting vibro-acoustic events $(e_{va})$ in which instants $(t_i)$ associated with the vibro-acoustic events $(e_{va})$ are identified in the signal obtained at the end of the filtering step 400).

2. The method according to claim 1, wherein step 300) comprises a first sub-step 310) of normalizing the recorded audio signal $(S_E)$ so as to obtain a normalized recorded audio signal $(S_{E,n})$.

3. The method according to claim 2, wherein step 300) comprises a second sub-step 320) in which the reference audio signal $(S_R)$ is resampled at the same rate as the recorded audio signal $(S_E)$ and normalized so as to obtain a resampled and normalized reference audio signal $(S_{R,n})$.

4. The method according to any of claims 2 to 3, wherein the normalization operation performed in step 300) is performed by means of a normalization function defined by:

$$f_n(X) := \frac{X - mean\,(X)}{median\,\big(abs(X)\big)}$$

   Where X is the recorded audio signal $(S_E)$ or the resampled reference audio signal $(S_R)$, mean (X) is the mean value of the signal under consideration, abs (X) is the signal whose samples are absolute values of the samples of the signal X and median (abs(X)) is the median value of the signal abs (X).

5. The method according to any of claims 3 to 4, wherein step 300) comprises, subsequent to the first and the second sub-step 320), a third sub-step 330) of identifying the beginning of the resampled and normalized reference audio signal $(S_{R,n})$ in the normalized recorded audio signal $(S_{E,n})$.

6. The method according to claim 5, wherein the third sub-step 330) comprises the following sub-steps:

   - 332) constructing a suitable filter so as to identify in the normalized recorded audio signal $(S_{E,n})$ the most probable sampling instant $(t_{SR})$ which corresponds to the beginning of the K first samples of the resampled and normalized reference audio signal $(S_{R,n})$,
   - 334) determining a time window of size $(T_{SR,n})$ in which searching for the normalized resampled reference audio signal $(S_{R,n})$ in the normalized recorded audio signal $(S_{E,n})$,
   - (336) determining the sampling instant $(t_{SR})$.

7. The method according to claim 6, wherein the sampling instant $(t_{SR})$ is defined as follows:

$$t_{SR} := argmax_{t < T_{SR,n}} \sum_{k=t}^{t+K} S_{E,n}(k) \cdot S_{R,n}(k - t)$$

Where (t) corresponds to a given sampling instant, ($t_{SR}$) expresses the sampling instant corresponding to the beginning of the resampled and normalized reference audio signal ($S_{R,n}$) in the normalized recorded audio signal ($S_{E,n}$), ($T_{SR,n}$) is the size of the time window (in seconds), and argmax, denotes the set of points, over the time window ($T_{SR,n}$), at which the convolution product $\sum_{k=t}^{t+K} S_{E,n}(k) \cdot S_{R,n}(k - t)$ reaches its maximum value.

8.  The method according to any one of the preceding claims, wherein the filtering step 400) comprises a first filtering sub-step 410) and optionally a second filtering sub-step 420).

9.  The method according to claim 8, wherein:

    - the first filtering sub-step 410) is implemented by means of a 1st order Butterworth type bandpass filter having a low frequency of 50 Hz and a high frequency of 200 Hz, and
    - the second filtering sub-step 420) is implemented by means of a 1st order Butterworth type low-pass filter having a high frequency of 400 Hz,

    so that at the end of the second sub-step 420) a filtered normalized recorded audio signal ($S_{E,n,f}$).

10. The method according to one of claims 8 to 9, wherein subsequent to the second sub-step 420), step 400) comprises a third sub-step 430) of local normalization of the filtered normalized recorded audio signal ($S_{E,n,f}$) obtained at the end of step 420) so as to obtain a filtered normalized recorded audio signal ( $\widehat{S_{E,n,f}}$ ), said local normalization sub-step 430) being carried out by means of a local normalization function defined by:

$$f_{nloc}(S_{E,n,f}) := \frac{S_{E,n,f} - mean_{loc}(S_{E,n,f})}{mean_{loc}(abs_{loc}(S_{E,n,f} - mean_{loc}(S_{E,n,f})))}$$

Where ($S_{E,n,f}$) is the filtered normalized recorded audio signal, $mean_{loc}(S_{E,n,f})$ is the local mean value of the filtered normalized recorded audio signal ($S_{E,n,f}$), $abs_{loc}(S_{E,n,f} - mean_{loc}(S_{E,n,f}))$ is the signal whose the samples are local absolute values of the samples of the filtered normalized recorded audio signal ($S_{E,n,f}$) over the subset of a sliding window of size $2T_{norm}$.

11. The method according to any one of claims 8 to 10, in which, subsequent to the filtering step 400), step 500) of detecting vibro-acoustic events ($e_{va}$) is implemented.

12. The method according to claim 11, wherein step 500) comprises a first sub-step 510) of determining the energy (of the filtered normalized recorded audio signal ( $\widehat{S_{E,n,f}}$ ), said first sub-step 510) being performed by means of an energy function defined by:

$$E_{\widehat{S_{E,n,f}}}(t) = \sum_{k=-T_E}^{T_E} \left| \widehat{S_{E,n,f}}(t + k) \right|^2$$

Where (t) corresponds to a given sampling instant, ($2T_E$) is the size of a sliding time window, the number of samples being equal to $2T_E$ in said sliding window, and ( $\widehat{S_{E,n,f}}$ ) is the filtered normalized recorded audio signal obtained at the end of step 430).

**13.** The method according to claim 12, wherein step 500) comprises, subsequent to the first sub-step 510), a second sub-step 520) of smoothing the signal ( $E_{\widehat{S_{E,n,f}}}$ ) obtained at the end of the first sub-step 510) by means of a smoothing function defined by the convolution product of the signal ( $E_{\widehat{S_{E,n,f}}}$ ) with a Hamming-type weighting window:

$$\tilde{E}_{\widehat{S_{E,n,f}}} := E_{\widehat{S_{E,n,f}}} * Hamming\ (T_{lissage})$$

Where ( $\tilde{E}_{\widehat{S_{E,n,f}}}$ ) is the smoothed signal, ( $E_{\widehat{S_{E,n,f}}}$ ) is the signal before smoothing, Hamming is the weighting window, and ($T_{smoothing}$) is the size of the weighting window.

**14.** The method according to claim 13, wherein step 500) comprises, subsequent to the second sub-step 520, a third sub-step 530) of extracting from the smoothed signal ( $'\tilde{E}_{\widehat{S_{E,n,f}}}$ ), the set of P sampling instants ($t_i$) corresponding to local maxima (mli) and/or onset candidates of vibro-acoustic events ($e_{va}$), said sampling instants being such that

$$\tilde{E}_{\widehat{S_{E,n,f}}}\ (t_i) > \tilde{E}_{\widehat{S_{E,n,f}}}\ (t_{i-1})$$

and

$$\tilde{E}_{\widehat{S_{E,n,f}}}\ (t_i) > \tilde{E}_{\widehat{S_{E,n,f}}}\ (t_{i+1})$$

**15.** The method according to claim 14, wherein step 500) comprises, subsequent to the third sub-step 530), a fourth sub-step 540) of pre-selecting the onset candidates of vibro-acoustic event ($e_{va}$) comprising the following sub-steps:

- 5400) grouping candidates ($m_{li}$) associated with the P sampling instants ($t_i$) according to a first selection criterion, said first selection criterion corresponding to the grouping of the candidate sampling instants spaced by a predetermined number m of samples, so as to form groups ($g_j$) of local maxima ($m_{li}$),
- 5401) conserving in each group $g_j$ the instants associated with the local maxima ($m_{li}$) for which the maximum value of $\tilde{E}_{\widehat{S_{E,n,f}}}$ is obtained.

**16.** The method according to claim 15, wherein step 500) comprises, subsequent to the fourth sub-step 540), a fifth sub-step 550) of removing the spurious maxima ($m_{li}$) comprising the following sub-steps:

- 5500) sorting, by decreasing height, the local maxima ($m_{li}$) kept at the end of the fourth sub-step 540),
- 5501) conserving the $\rho N_{tap}$ largest local maxima ($m_{li}$), $N_{tap}$ being the number of vibro-acoustic events ($e_{va}$) comprised in the measurement time window and $\rho$ being strictly greater than 1, $\rho > 1$.

**17.** The method according to claim 16, further comprising, subsequent to step 500) of detecting vibro-acoustic events ($e_{va}$), an additional step 600) of optimizing the signal obtained at the end of step 500), said step comprising the following sub-steps:

- 610) pairing the set of the instants ($t_i$), with $i < \rho N_{tap}$, of the local maxima ($m_{li}$) kept in step 5502) with the set of model instants ($t_j$), with $j < N_{tap}$, measured by the touchscreen (10),
- 620) evaluating the quality of the pairing performed during the sub-step 610) by means of an objective function ($f(\delta)$),
- 630) maximizing the objective function ($f(\delta)$) by means of a parameter ($\delta^{opt}$),
- 640) selecting the local maxima ($m_{li}$) associated with the sampling instants ($t_i$) which are exactly paired to the model instants ($t_j$) measured by the touchscreen.

**18.** The method according to claim 17, wherein the objective function is defined by:

$$f(\delta) := \left| \left\{ j \in [1, N_{tap}] \, t. \, q. \quad matches(\tilde{t}_j, \delta) = 1 \right\} \right|$$

Where the function $matches(\tilde{t}_j, \delta)$ is defined by:

$$matches(\tilde{t}_j, \delta) := \left| \left\{ i \in [1, \rho N_{tap}] \, t. \, q. \quad \tilde{t} + \rho - t_i < \epsilon \right\} \right|$$

and ($\varepsilon$) is a threshold value (in milliseconds) that controls the maximum difference tolerated to consider the pairing is of quality.

**19.** The method according to any of claims 17 to 18, wherein the parameter ($\delta^{opt}$) is defined by:

$$\delta^{opt} := argmax_{\delta \in [0, \delta_{max}]} f(\delta)$$

Where $\delta_{max}$ is an appropriate threshold value below $\tilde{t} N_{tap}$.

**20.** The method according to any one of claims 17 to 19, wherein step 600) comprises, subsequent to the fourth sub-step 640), a fifth adjusting sub-step 650) during which the local maxima $(m_{li})$ selected at the end of step 640) are adjusted so as to conform to the recorded audio signal $(S_E)$.

**21.** The method according to claim 20, wherein the adjustment is performed by applying a phase shift compensation function to the maxima $m_{li} = \tilde{E}_{\widehat{S_{E,n,f}}}(t_i)$, said function being defined by:

$$\widehat{t_{m_{li}}} := argmax_{t \in [t_i - T_i, \ t_i + T_i]} E_{\widehat{S_{E,n,f}}}(t_i)$$

Where ($t_i$) is the sampling instant associated with the local maxima mli selected at the end of step 560), ($T_i$) being the number of samples comprised in the search time window, ($E_{\widehat{S_{E,n,f}}}$) is the signal before smoothing.

**22.** A touchscreen device (1) suitable for carrying out the method according to any one of claims 1 to 21, said device comprising:

- a touchscreen (10),
- a microphone (30),
- a central processing unit configured to perform at least steps of the method according to any of claims 1 to 21.

**23.** A computer program comprising instructions which when the program is executed by the computer cause the computer to carry out the method of any one of claims 1 to 21.

**24.** A computer storage medium on which the computer program according to claim 23 is recorded.

EP 3 899 701 B1

*Fig. 1*

Fig. 2

```
                                                    ⌒(100)
┌─────────────────────────────────────────┐
│   Lecture du signal audio de référence (S_R) │
└─────────────────────────────────────────┘
                    │
                    ▼                       ⌒(200)
┌─────────────────────────────────────────┐
│  Enregistrement du signal audio de référence S_R │
│     et d'un ou plusieurs évènements vibro-      │
│   acoustique e_va, obtention du signal audio    │
│              enregistré S_E                     │
└─────────────────────────────────────────┘
                    │
                    ▼                       ⌒(300)
┌─────────────────────────────────────────┐
│  Détection du signal audio de référence S_R dans │
│        le signal audio enregistré S_E           │
└─────────────────────────────────────────┘
                    │
                    ▼                       ⌒(400)
┌─────────────────────────────────────────┐
│     Filtrage du signal enregistré obtenu        │
│    subséquemment à l'étape (300) et             │
│              normalisation                      │
└─────────────────────────────────────────┘
                    │
                    ▼                       ⌒(500)
┌─────────────────────────────────────────┐
│   Détection des évènements vibro-acoustiques    │
│   (e_va) dans le signal obtenu à la fin de l'étape │
│                   (400)                         │
└─────────────────────────────────────────┘
                    │
                    ▼                       ⌒(600)
┌─────────────────────────────────────────┐
│  Optimisation du signal obtenu à la fin de l'étape │
│                   (500)                         │
└─────────────────────────────────────────┘
```

# Fig. 3

(300)

(310)

Normalisation du signal audio enregistré ($S_E$)

(320)

Ré-échantillonnage du signal audio de référence ($S_R$) au même taux que le signal audio enregistré ($S_E$) et normalisation

(330)

Identification du début du signal audio de référence ré-échantillonné et normalisé ($S_{R,n}$) dans le signal audio enregistré normalisé ($S_{E,n}$)

(332)

Construction d'un filtre adapté

(334)

Détermination d'une fenêtre temporelle de taille ($T_{SR,n}$) dans laquelle effectuer la recherche du signal audio de référence ré-échantillonné normalisé ($S_{R,n}$) dans le signal audio enregistré normalisé ($S_{E,n}$)

(336)

Détermination de l'instant d'échantillonnage ($t_{SR}$)

Fig. 4

31

(400)

(410)

Première sous-étape de filtrage : utilisation
d'un filtre passe-bande de type Butterworth
d'ordre 1 ayant une fréquence basse de 50 Hz
et une fréquence haute de 200 Hz

(420)

Deuxième sous-étape de filtrage : utilisation
d'un filtre passe-bas de type Butterworth
d'ordre 1 ayant une fréquence haute de 400 Hz

(430)

Normalisation locale du signal audio enregistré
normalisé filtré ($S_{E,n,f}$) obtenu à la fin de l'étape
(420)

Fig. 5

(500)

(510)

Détermination de l'énergie $E_{\widehat{S_{E,n,f}}}$ du signal audio enregistré normalisé filtré ($\widehat{S_{E,n,f}}$)

(520)

Lissage du signal $E_{\widehat{S_{E,n,f}}}$ obtenu à la fin de la première sous-étape (510)

(530)

Extraction à partir du signal lissé $\tilde{E}_{\widehat{S_{E,n,f}}}$ de l'ensemble des P instants d'échantillonnage $t_i$ correspondant à des maxima locaux $m_{li}$

(540)

Présélection

(5400)

Regroupement de candidats $m_{li}$ associés aux P instants d'échantillonnage $t_i$ selon un premier critère de sélection, formation de groupes $g_j$ de maxima locaux $m_{li}$,

(5401)

Conservation dans chaque groupe $g_j$ des maxima locaux $m_{li}$ pour lesquels la valeur maximale de $\tilde{E}_{\widehat{S_{E,n,f}}}$ est obtenue

(550)

Elimination des maxima $m_{li}$ parasites

Fig. 6

(500)

(510-540)

....

(550)

Elimination des maxima m$_{li}$ parasites

(5500)

Tri, par hauteur décroissante, des maxima locaux m$_{li}$ conservés à la fin de l'étape (540)

(5501)

Conservation des $\rho N_{tap}$ plus grands maxima locaux m$_{li}$, N$_{tap}$ étant le nombre d'évènements vibro-acoustique ($e_{va}$) pendant la fenêtre temporelle de mesure et $\rho$ étant strictement supérieur à 1, $\rho > 1$

Fig. 7

(600)

(610)

Appariement de l'ensemble des instants $t_i$, avec $i < \rho N_{tap}$, des maxima locaux $m_{li}$ conservés à l'étape (5501) avec l'ensemble des instants modèles $t_j$, avec $j < N_{tap}$, mesurés par l'écran tactile (10)

(620)

Evaluation de la qualité de l'appariement effectué lors de la sous-étape (610) au moyen d'une fonction objectif $f(\delta)$,

(630)

Maximisation de la fonction objectif $f(\delta)$ au moyen d'un paramètre $\delta^{opt}$

(640)

Sélection des maxima locaux $m_{li}$ associés aux instants d'échantillonnage $t_i$ qui sont appariés exactement aux instants modèles $t_j$ mesurés par l'écran tactile

(650)

Ajustement des maxima locaux $m_{li}$ sélectionnés à l'issu de l'étape (640) de sorte qu'ils se conforment au signal audio enregistré ($S_E$)

Fig. 8

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20140249447 A
- EP 2875419 A
- WO 2005122898 A1